# EUROPÄISCHE PATENTSCHRIFT

(11) **EP 4 290 234 B1**
(45) Veröffentlichungstag und Bekanntmachung des Hinweises auf die Patenterteilung: **28.10.2026**
(21) Anmeldenummer: 23175964.8
(22) Anmeldetag: 30.05.2023
(51) Int. Cl.: G01N 33/497, A61B 5/097

(54) **ANALYSEGERÄT MIT EINEM MOTOR UND EINER NOCKENSCHEIBE UND ANALYSEVERFAHREN MIT EINEM SOLCHEN ANALYSEGERÄT**
ANALYZER COMPRISING A MOTOR AND A CAM DISC AND ANALYZING METHOD USING SUCH AN ANALYZER
ANALYSEUR AVEC UN MOTEUR ET UN DISQUE À CAMES ET PROCÉDÉ D'ANALYSE UTILISANT UN TEL ANALYSEUR

(30) Priorität: 08.06.2022 DE 102022114344
(43) Veröffentlichungstag der Anmeldung: 13.12.2023
(73) Patentinhaber: Dräger Safety AG & Co. KGaA, 23560 Lübeck (DE)
(72) Erfinder: Baesler, Malte, 23558 Lübeck (DE); Brunswick-Franco, Luis, 23558 Lübeck (DE); Reier, Tobias, 23558 Lübeck (DE); Rekow, Jens, 23558 Lübeck (DE)
(74) Vertreter: Meyer-Gramann, Klaus Dieter

(56) Entgegenhaltungen:
- CN-A- 114 460 030
- DE-A1- 2 702 516
- US-A- 5 117 674

## Beschreibung

Die Erfindung betrifft ein Analysegerät und ein Analyseverfahren zum Analysieren eines Gases auf mindestens eine vorgegebene Substanz.

Ein solches Analysegerät lässt sich beispielsweise für die Überprüfung verwenden, ob ein Proband Alkohol, insbesondere Äthylalkohol (Ethanol) und optional Beimischungen anderer Alkohole, zu sich genommen hat oder nicht. Wenn der Proband Alkohol zu sich genommen hat und dieser Alkohol noch nicht vollständig abgebaut wurde, so enthält sein Blut Alkohol und daher die vom Probanden ausgeatmete Luft Atemalkohol. Optional lässt sich mit einem solchen Analysegerät feststellen, wie groß die Atemalkohol-Konzentration in seinem Atem ist, und daraus lässt sich der Gehalt von Alkohol in seinem Blut ableiten. Das Analysegerät untersucht eine vom Probanden abgegebene, insbesondere eine vom Probanden ausgeatmete Atemprobe.

Im Folgenden wird die Bezeichnung "Alkohol" für eine zu detektierende Substanz im Blut des Probanden verwendet, die Bezeichnung "Atemalkohol" für eine Substanz, die dann in einer Gasprobe, insbesondere Atemprobe, des Probanden enthalten ist, wenn sein Blut Alkohol enthält.

DE 27 02 516 A1 offenbart ein Gasanalysegerät mit einem Sensor in einer Messkammer, die über einen Einlass mit der Umgebung in Verbindung steht. Eine ventillose Ansaugkammer 24 mit veränderbarem Volumen lässt sich über eine Stange 26 und eine Kurbelscheibe 28 ausdehnen und zusammenziehen. Die Kurbelscheibe 28 lässt sich durch eine Nockenscheibe ersetzen.

In DE 39 04 994 A1 wird ein Nachweisgerät 1 zur Bestimmung des Bestandteils einer Komponente eines Gases, insbesondere des Anteils von Alkohol in der Atemluft, beschrieben. Ein Zylinder 9 umgibt eine Vorkammer 12, die als Messkammer fungiert, sowie einen Hubraum 15. Ein Kolben 8 im Zylinder 9 begrenzt auf einer Seite den Hubraum 15 und ist über eine Pleuelstange 20 mit einem Exzenter 16 verbunden. Ein Motorantrieb 10 dreht den Exzenter 16 und bewirkt dadurch, dass der Kolben 8 im Zylinder 9 hin und her gleitet. Der Kolben 8 führt zunächst einen Ansaughub aus, bei dem der Hubraum 15 vergrößert wird und dadurch eine Gasprobe angesaugt wird, und anschließend einen Ausblashub, bei dem der Hubraum 15 wieder verkleinert wird und dadurch eine Gasprobe wieder ausgestoßen wird.

CN 114460030 A offenbart ein Gasanalysegerät mit Messkammer, Ansaugkammer, Motor, Nockenscheibe, Betätigungselement und Einlassventil.

Der Erfindung liegt die Aufgabe zugrunde, ein Analysegerät und ein Analyseverfahren zum Untersuchen eines Gases auf eine vorgegebene Substanz bereitzustellen, wobei das Analysegerät und das Analyseverfahren zuverlässiger als bekannte Analysegeräte und Analyseverfahren sein sollen.

Die Aufgabe wird durch ein Analysegerät mit den Merkmalen des Anspruchs 1 und durch ein Analyseverfahren mit den Merkmalen des Anspruchs 11 gelöst. Vorteilhafte Ausgestaltungen des erfindungsgemäßen Analysegeräts sind, soweit sinnvoll, auch vorteilhafte Ausgestaltungen des erfindungsgemäßen Analyseverfahrens und umgekehrt.

Das erfindungsgemäße Analysegerät und das erfindungsgemäße Analyseverfahren vermögen ein Gas auf mindestens eine vorgegebene Substanz zu analysieren.

Das erfindungsgemäße Analysegerät umfasst eine Messkammer. Diese Messkammer steht wenigstens zeitweise in einer einströmseitigen Fluidverbindung mit einer Umgebung des Analysegeräts. Durch diese einströmseitige Fluidverbindung hindurch kann Gas, das auf die Substanz untersucht werden soll, aus der Umgebung in die Messkammer fließen. Dadurch wird in der Messkammer eine Gasprobe gebildet.

Weiterhin umfasst das Analysegerät einen Sensor. Der Sensor vermag ein Maß für die Menge und / oder die Konzentration der Substanz in einer Gasprobe zu messen, wobei diese Gasprobe sich in der Messkammer befindet.

Eine Ansaug-Kammereinheit des Analysegeräts weist ein veränderbares Volumen auf und steht in einer ausströmseitigen Fluidverbindung mit der Messkammer. Durch diese ausströmseitige Fluidverbindung hindurch kann Gas aus der Messkammer in die Ansaug-Kammereinheit und umgekehrt aus der Ansaug-Kammereinheit in die Messkammer fließen. Die Ansaug-Kammereinheit kann insbesondere einen Balg und eine Platte oder eine Kolben-Zylinder-Einheit umfassen.

Der Schritt, das Volumen der Ansaug-Kammereinheit zu vergrößern, bewirkt folgendes: Gas wird aus der Umgebung des Analysegeräts durch die einströmseitige Fluidverbindung hindurch in die Messkammer gesaugt. Der Schritt, das Volumen der Ansaug-Kammereinheit zu verkleinern, bewirkt folgendes: Gas wird aus der Messkammer ausgestoßen und in die Umgebung gefördert. Das aus der Messkammer ausgestoßene Gas wird bevorzugt durch die einströmseitige Fluidverbindung hindurch in die Umgebung gefördert, alternativ durch eine separate Fluidverbindung.

Eine Betätigungseinheit des Analysegeräts lässt sich relativ zur Messkammer in eine erste Richtung und in eine zweite Richtung bewegen, bevorzugt linear bewegen. Die zweite Richtung ist der ersten Richtung entgegengesetzt. Die Betätigungseinheit ist mit der Ansaug-Kammereinheit mechanisch verbunden. Der Schritt, die Betätigungseinheit in die erste Richtung zu bewegen, bewirkt folgendes: Das Volumen der Ansaug-Kammereinheit wird vergrößert. Der Schritt, die Betätigungseinheit in die zweite Richtung zu bewegen, bewirkt folgendes: Das Volumen der Ansaug-Kammereinheit wird verkleinert.

Eine Nockenscheibe des Analysegeräts ist um eine Achse drehbar. Ein Motor des Analysegeräts vermag die Nockenscheibe um diese Achse zu drehen.

Die Betätigungseinheit ist mit der Nockenscheibe mechanisch gekoppelt, und zwar nach Art eines Nockenfolgers. Die Betätigungseinheit steht wenigstens zeitweise, bevorzugt dauerhaft in einem Kontakt mit der Nockenscheibe, wobei dieser Kontakt lösbar ist.

Diese mechanische Kopplung bewirkt folgendes: Jede Rotationsposition der drehbaren Nockenscheibe relativ zur Achse legt eine Position der Betätigungseinheit fest, und zwar zumindest dann, wenn die Betätigungseinheit die Nockenscheibe berührt. Diese Position der Betätigungseinheit legt wiederum ein Volumen der Ansaug-Kammereinheit fest.

Das erfindungsgemäße Verfahren wird unter Verwendung eines solchen Analysegeräts durchgeführt und umfasst die folgenden Schritte:
- Wenigstens zeitweise ist die Messkammer durch die einströmseitige Fluidverbindung hindurch mit einer Umgebung des Analysegeräts verbunden. Das zu untersuchende Gas stammt aus dieser Umgebung.
- Wenigstens zeitweise dreht der Motor die Nockenscheibe um die Achse.
- Die mechanische Kopplung zwischen der Nockenscheibe und der Betätigungseinheit bewirkt, dass jede Rotationsposition der Nockenscheibe relativ zur Achse jeweils eine Position der Betätigungseinheit und damit ein Volumen der Ansaug-Kammereinheit festlegt.
- Die Betätigungseinheit wird in die erste Richtung bewegt. Dieser Schritt vergrößert das Volumen der Ansaug-Kammereinheit. Gas wird in die Ansaug-Kammereinheit hinein gesaugt.
- Diese Vergrößerung bewirkt, dass Gas aus der Umgebung durch die einströmseitige Fluidverbindung hindurch in die Messkammer gesaugt wird. Dadurch gelangt eine Gasprobe in die Messkammer.
- Der Sensor misst ein Maß für die Menge und / oder die Konzentration der Substanz in der Gasprobe in der Messkammer.
- Die Betätigungseinheit wird in die zweite Richtung bewegt. Dieser Schritt verkleinert das Volumen der Ansaug-Kammereinheit. Gas wird aus der Ansaug-Kammereinheit ausgestoßen.
- Diese Verkleinerung bewirkt, dass Gas aus der Messkammer ausgestoßen wird. Dadurch wird die Messkammer gespült.

Die Schritte des erfindungsgemäßen Verfahrens werden nicht notwendigerweise in der Reihenfolge dieser Beschreibung durchgeführt.

Erfindungsgemäß wird dadurch eine Gasprobe in die Messkammer gefördert, dass das Volumen der Ansaug-Kammereinheit vergrößert wird. Dadurch, dass das Volumen der Ansaug-Kammereinheit vergrößert wird, wird Gas aus der Messkammer durch die ausströmseitige Fluidverbindung hindurch in die Ansaug-Kammereinheit gesaugt. Durch die Konstruktion des Analysegeräts ist bekannt, um welchen Wert das Volumen vergrößert wird. Dadurch ist auch wenigstens näherungsweise bekannt, welches Volumen die Gasprobe aufweist, die aus der Umgebung in die Messkammer gefördert wird. Dieses Merkmal vergrößert die Zuverlässigkeit, mit welcher der Sensor die Menge und / oder die Konzentration der Substanz misst, verglichen mit einer Ausgestaltung, bei der Gas aus der Umgebung im Wesentlichen durch Diffusion in die Messkammer gelangt. Durch das Ansaugen wird außerdem die Messkammer schneller als bei einem Diffundieren mit einer Gasprobe gefüllt.

Erfindungsgemäß wird die Messkammer dadurch gespült, dass das Volumen der Ansaug-Kammereinheit verkleinert wird. Dadurch, dass das Volumen verkleinert wird, fließt Gas aus der Ansaug-Kammereinheit durch die ausströmseitige Fluidverbindung hindurch in die Messkammer. Nicht erforderlich ist eine separate Fluidfördereinheit, um die Messkammer auszuspülen. Nach dem Ausspülen ist das Analysegerät dafür bereit, eine weitere Gasprobe zu untersuchen, wobei die weitere Gasprobe sich in der Messkammer befindet. Die Gefahr ist gering, dass die alte Messprobe die Analyse der weiteren Messprobe verfälscht.

Eine bewegliche Betätigungseinheit vermag das Volumen der Ansaug-Kammereinheit zu vergrößern und zu verkleinern. Erfindungsgemäß umfasst das Analysegerät einen Antrieb, wobei dieser Antrieb die Betätigungseinheit in beide Richtungen zu bewegen und dadurch das Volumen der Ansaug-Kammereinheit zu vergrößern und zu verkleinern vermag.

Denkbar wäre, dass der Antrieb direkt die Betätigungseinheit hin und her zu bewegen vermag. Erfindungsgemäß ist der Antrieb hingegen über die drehbare Nockenscheibe mit der Betätigungseinheit verbunden. Der Antrieb ist als ein Motor ausgestaltet, wobei der Motor die Nockenscheibe um die Achse zu drehen vermag. Unter einem "Motor" wird ein Antrieb verstanden, der ein Bauteil über eine nicht konstruktionsbedingt beschränkte Strecke zu bewegen vermag - im Unterschied etwa zu einem Stellantrieb oder einer Kolben-Zylinder-Einheit. Ausreichend ist, dass der Motor die Nockenscheibe stets in eine Richtung zu drehen vermag, bevorzugt in einem Start-Stopp-Betrieb.

Ein Antrieb, der ein Bauteil - hier: die Nockenscheibe - mit einem ausreichend großen Drehmoment zu drehen vermag, lässt sich häufig mit einem relativ kleinen Bauraum realisieren - verglichen mit einem Stellantrieb. In vielen Fällen würde ein Antrieb, der direkt die Betätigungseinheit hin und her zu bewegen vermag, einen größeren Bauraum und / oder mehr elektrische Energie erfordern.

Erfindungsgemäß ist die Betätigungseinheit mechanisch mit der drehbaren Nockenscheibe gekoppelt. Die Betätigungseinheit fungiert als ein Nockenfolger. Jede Rotationsposition der Nockenscheibe legt jeweils eine Position der Betätigungseinheit und damit jeweils ein Volumen der Ansaug-Kammereinheit fest - zumindest dann, wenn die Betätigungseinheit die Nockenscheibe berührt. Der zeitliche Verlauf des Vorgangs, die Nockenscheibe zu drehen, sowie die Geometrie der Nockenscheibe legen daher den zeitlichen Verlauf des variierenden Volumens der Ansaug-Kammereinheit fest. Die zeitliche Veränderung des Volumens der Ansaug-Kammereinheit legt den Vorgang fest, dass und wie viel und wie schnell Gas in die Messkammer gesaugt wird. Die Erfindung erleichtert es daher, einen vorgegebenen Ablauf beim Füllen und Ausspülen der Messkammer zu realisieren. Insbesondere ist es leichter möglich zu bewirken, dass die Menge und / oder das Volumen der Gasprobe, die sich tatsächlich in der Messkammer befindet, bis auf eine Toleranz gleich einer vorgegebenen Menge oder einem vorgegebenen Volumen ist.

Erfindungsgemäß ist zwischen der Betätigungseinheit und der Nockenscheibe ein mechanischer Kontakt hergestellt. Die Betätigungseinheit fungiert als ein Nockenfolger und gleitet dann, wenn die Nockenscheibe gedreht wird, über die Oberfläche der Nockenscheibe. In der Regel lässt sich dieser mechanische Kontakt lösen, und zwar dadurch, dass die Betätigungseinheit räumlich von der Nockenscheibe entfernt wird. Diese Ausgestaltung erleichtert es, das Analysegerät zusammenzubauen, zu reinigen und zu reparieren, verglichen z.B. mit einer Ausgestaltung, bei der die Betätigungseinheit mit Hilfe eines Exzenters und einer Kolben-Zylinder-Einheit realisiert ist.

Bevorzugt legt eine erste Rotationsposition der Nockenscheibe ein maximales Volumen der Ansaug-Kammereinheit fest. Eine zweite Rotationsposition legt ein minimales Volumen fest. Das minimale Volumen ist kleiner als das maximale Volumen. Die beiden Rotationspositionen unterscheiden sich voneinander.

Bevorzugt ist eine mechanische oder pneumatische Feder des Analysegeräts bestrebt, die Betätigungseinheit gegen die Nockenscheibe zu drücken. Diese Feder kann insbesondere als Druckfeder oder Zugfeder ausgestaltet sein. Dank der Feder wird die mechanische Kopplung zwischen der Nockenscheibe und der Betätigungseinheit hergestellt. Mindestens aber trägt die Feder zu dieser mechanischen Kopplung bei.

Im laufenden Betrieb kann das Ereignis auftreten, dass die Betätigungseinheit den Kontakt zur Nockenscheibe verliert, beispielsweise aufgrund von Erschütterungen oder Vibrationen oder einer schnellen Beschleunigung des Analysegeräts. Auch in diesem Falle stellt die Feder den mechanischen Kontakt in der Regel rasch wieder her.

In einer Realisierungsform liegen die Nockenscheibe, die Betätigungseinheit und die Messkammer in einer Linie. Bevorzugt erstreckt sich die Betätigungseinheit entlang dieser Linie. Die Ausgestaltung, dass diese drei Bestandteile in einer Linie liegen, spart in vielen Fällen Platz ein und führt insbesondere zu einer relativ geringen Abmessung des Analysegeräts in eine Richtung senkrecht zu dieser Linie.

Erfindungsgemäß vermag der Motor die Nockenscheibe um die Achse zu drehen. Verschiedene Ausgestaltungen der Nockenscheibe sind möglich.

In einer Ausgestaltung weist die Nockenscheibe eine Umfangskontur auf. Diese Umfangskontur weicht von einer Kreisform ab. Genauer gesagt: Der Abstand zwischen der Umfangskontur und der Achse, um welche die Nockenscheibe drehbar ist, variiert entlang der Umfangskontur. Sowohl die erste Richtung als auch die zweite Richtung, in welche die Betätigungseinheit relativ zur Messkammer beweglich ist, stehen senkrecht auf der Achse, um welche die Nockenscheibe drehbar ist. Die Betätigungseinheit berührt diese Umfangskontur, und die Betätigungseinheit wird wenigstens zeitweise bewegt, wenn die Nockenscheibe mit der variierenden Umfangskontur gedreht wird. Die Ausgestaltung mit der variierenden Umfangskontur ermöglicht es, den Motor seitlich von der beweglichen Betätigungseinheit anzuordnen. Die Nockenscheibe kann eine relativ geringe Abmessung in die beiden Richtungen aufweisen, in welche die Betätigungseinheit beweglich ist.

Diese Ausgestaltung lässt sich kombinieren mit der Ausgestaltung, bei der eine Feder bestrebt ist, die Betätigungseinheit gegen die Nockenscheibe zu drücken. Bei dieser Kombination ist die Feder bestrebt, die Betätigungseinheit gegen die Umfangskontur der Nockenscheibe zu bewegen, also in eine Richtung senkrecht zur Achse der Nockenscheibe.

In einer alternativen Ausgestaltung weist die Nockenscheibe eine Flächenkontur auf. Diese Flächenkontur erstreckt sich in einer Ebene, wobei diese Ebene senkrecht auf der Achse steht, um welche die Nockenscheibe drehbar ist. Die Flächenkontur der Nockenscheibe weicht von einer ebenen Form ab. Genauer gesagt: Der Abstand zwischen der Flächenkontur und der Ebene variiert über die Flächenkontur. Die beiden Richtungen, in welche die Betätigungseinheit relativ zur Messkammer beweglich ist, sind parallel zur Achse, um welche die Nockenscheibe drehbar ist, und stehen senkrecht auf der gerade genannten Ebene. Die Ausgestaltung mit der variierenden Flächenkontur führt in vielen Fällen dazu, dass die Nockenscheibe eine kleinere Ausdehnung in eine Richtung parallel zur Achse aufweist.

Auch diese Ausgestaltung lässt sich mit der Ausgestaltung umfassend eine Feder kombinieren. Bei dieser Kombination ist die Feder bestrebt, die Betätigungseinheit gegen die veränderliche Flächenkontur zu bewegen, also parallel zur Achse der Nockenscheibe.

Erfindungsgemäß bewirkt eine volle Umdrehung der Nockenscheibe um die Achse, dass die Betätigungseinheit mindestens einmal in die erste Richtung und anschließend mindestens einmal in die zweite Richtung bewegt wird. Bei der oder jeder Bewegung in die erste Richtung wird das Volumen der Ansaug-Kammereinheit vergrößert. In einer Ausgestaltung ist die Nockenscheibe so ausgestaltet, dass bei einer vollen Umdrehung der Nockenscheibe um die Achse die Betätigungseinheit zweimal hintereinander in die erste Richtung bewegt wird. Zwischen diesen beiden Bewegungen in die erste Richtung wird die Betätigungseinheit in die zweite Richtung bewegt. Dadurch wird das Volumen der Ansaug-Kammereinheit zuerst vergrößert, dann verkleinert und dann wieder vergrößert.

Die Ausgestaltung, bei der die Betätigungseinheit bei einer vollen Umdrehung zweimal hintereinander in die erste Richtung bewegt wird, ermöglicht es, dass das Analysegerät während einer einzigen Umdrehung der Nockenscheibe zweimal hintereinander jeweils eine Gasprobe einsaugt. Insbesondere die Geometrie der Nockenscheibe legt fest, welches Volumen die Gasprobe hat, die beim ersten Mal eingesaugt wird, und welches Volumen die beim zweiten Mal eingesaugte Gasprobe hat. Die beiden Gasproben können insbesondere unterschiedliche Volumina aufweisen, und die beiden Vorgänge, diese beiden Gasproben einzusaugen, können unterschiedlich lange dauern. Zwischendurch wird die Messkammer gespült.

Eine mögliche Anwendung der Ausgestaltung, bei der das Volumen der Ansaug-Kammereinheit zuerst vergrößert, dann verkleinert und dann wieder vergrößert wird, ist die folgende: Die Atemluft eines Probanden soll auf Alkohol untersucht werden. Zunächst soll Atemluft aus der Mund des Probanden untersucht werden, um festzustellen, ob der Proband kürzlich Alkohol getrunken hat. Anschließend soll Atemluft aus der Lunge des Probanden untersucht werden, um festzustellen, ob das Blut des Probanden Alkohol enthält. Atemluft aus den oberen Atemwegen des Probanden soll hingegen nicht untersucht werden und soll daher idealerweise nicht in die Messkammer gelangen.

Erfindungsgemäß kann Gas durch die einströmseitige Fluidverbindung hindurch in die Messkammer fließen. Erfindungsgemäß lässt sich ein Ventil des Analysegeräts zwischen einer verschließenden und einer freigebenden Endposition hin und her bewegen. In der verschließenden Endposition verschließt das Ventil die einströmseitige Fluidverbindung. In der freigebenden Endposition gibt das Ventil die einströmseitige Fluidverbindung frei. Bevorzugt gibt das Ventil auch in jeder Zwischenposition die einströmseitige Fluidverbindung frei, aber mit einer geringeren Querschnittsfläche, die zum Fließen von Gas zur Verfügung steht, verglichen mit der freigebenden Endposition. In der verschließenden Endposition sowie in jeder Zwischenposition ist einerseits die Gefahr reduziert oder sogar ausgeschlossen, dass Schadgase oder Partikel in der Umgebung in die Messkammer gelangen und damit den Sensor erreichen. Andererseits ist die Gefahr reduziert oder sogar ausgeschlossen, dass ein Bestandteil des Sensors verdunstet. In jeder Zwischenposition sowie in der freigebenden Endposition kann Gas in die Messkammer fließen, und ein Druckausgleich zwischen der Umgebung und der Messkammer kann stattfinden. Bevorzugt umfasst das Ventil einen Ventilkörper und einen Ventilkörper-Sitz, wobei der Ventilkörper relativ zum Ventilkörper-Sitz beweglich ist.

Gas kann nur dann aus der Umgebung in die Messkammer fließen, wenn das Ventil in der freigebenden Endposition oder in einer Zwischenposition ist. Möglich ist, dass das Ventil einen eigenen Antrieb umfasst, welcher das Ventil zwischen den beiden Endpositionen hin und her zu bewegen vermag.

In einer bevorzugten Ausgestaltung ist hingegen das Ventil, mindestens aber der Ventilkörper, mit der Betätigungseinheit mechanisch verbunden. Dadurch vermag der Motor mittels der Nockenscheibe nicht nur das Volumen der Ansaug-Kammereinheit zu verändern, sondern zusätzlich das Ventil aus der einen Endposition in die andere Endposition zu bewegen. Diese Ausgestaltung spart einen separaten Antrieb für das Ventil ein.

Bevorzugt sind die Ansaug-Kammereinheit und das Ventil mittels der Betätigungseinheit wie folgt miteinander gekoppelt:
- In einer ersten Alternative bewirkt der Vorgang, dass die Betätigungseinheit in die erste Richtung bewegt wird, dass das Ventil in Richtung der verschließenden Endposition bewegt wird. Der Vorgang, dass die Betätigungseinheit in die zweite Richtung bewegt wird, bewirkt, dass das Ventil in Richtung der freigebenden Endposition bewegt wird.
- In einer zweiten Alternative bewirkt der Vorgang, dass die Betätigungseinheit in die erste Richtung bewegt wird, dass das Ventil in Richtung der freigebenden Endposition bewegt wird. Der Vorgang, dass die Betätigungseinheit in die zweite Richtung bewegt wird, bewirkt, dass das Ventil in Richtung der verschließenden Endposition bewegt wird.

Diese Synchronisation ermöglicht oft mit besonders hoher Zuverlässigkeit, dass die einströmseitige Fluidverbindung nur so lange wie nötig geöffnet und so lange wie möglich geschlossen oder wenigstens weitgehend verschlossen ist. Außerdem wird der zeitliche Verlauf der Bewegung des Ventils mit dem zeitlichen Verlauf des Volumens der Ansaug-Kammereinheit synchronisiert.

In einer Ausgestaltung umfasst die Betätigungseinheit eine starre Stange und ein Kontaktelement. Das Kontaktelement steht in Kontakt mit der Nockenscheibe. In einer Realisierungsform ist die oben beschriebene Feder bestrebt, das Kontaktelement gegen die Nockenscheibe zu drücken. Die Stange verbindet das Kontaktelement mechanisch mit dem Ventil. Diese Ausgestaltung führt in vielen Fällen zu einer besonders platzsparenden und robusten Konstruktion des Analysegeräts.

In einer Ausgestaltung dreht der Motor die Nockenscheibe so, dass die Nockenscheibe wenigstens eine volle Umdrehung um die Achse ohne eine Unterbrechung durchführt.

In einer Ausgestaltung wird die Drehung der Nockenscheibe gesteuert (openloop control), beispielsweise so, dass die Drehgeschwindigkeit gemäß einem vorgegebenen zeitlichen Verlauf der Nockenscheibe in einer Anlaufphase bis auf einen Maximalwert steigt, dann konstant auf diesem Maximalwert bleibt und in einer Endphase wieder reduziert wird, insbesondere auf Null. Bei dieser Ausgestaltung hängt die Position der Betätigungseinheit von der bislang verstrichenen Zeit seit dem Beginn der Drehung ab.

In einer bevorzugten Ausgestaltung wird die Drehung der Nockenscheibe hingegen geregelt (closed-loop control). Ein Positionssensor des Analysegeräts vermag automatisch ein Maß für die aktuelle Rotationsposition relativ zur Achse der Nockenscheibe relativ zu einer Referenz-Rotationsposition zu messen. Besonders bevorzugt lässt der Motor sich ansteuern und dadurch aktivieren (einschalten) und deaktivieren (ausschalten). Ein signalverarbeitendes Steuergerät (control unit) des Analysegeräts vermag den Motor anzusteuern und dadurch zu aktivieren und zu deaktivieren. Dieses Steuergerät vermag ein Signal des Positionssensors zu empfangen. Abhängig von diesem Signal des Positionssensor vermag das Steuergerät den Motor zu aktivieren und wieder zu deaktivieren.

Diese Ausgestaltung ermöglicht es, das Volumen der Ansaug-Kammereinheit abhängig von externen Ereignissen zu verändern, beispielsweise von einem Volumenfluss in die oder aus der Messkammer und / oder von einem Signal des Sensors in oder an der Messkammer. Das Ereignis kann auch daraus bestehen, dass ein Mundstück auf das Analysegerät aufgesetzt wird und das Analysegerät nunmehr bereit ist, eine Atemprobe aufzunehmen.

In einer Ausgestaltung umfasst der Positionssensor eine Lochscheibe und eine Lichtschranke. Die Lochscheibe kann sich um dieselbe Achse wie die Nockenscheibe drehen und ist drehfest mit der Nockenscheibe verbunden. Die Lochscheibe unterbricht einen Lichtstrahl der Lichtschranke. Mindestens eine Aussparung in der Lochscheibe gibt diesen Lichtstrahl frei. Außerhalb der oder jeder Aussparung unterbricht die Lochscheibe den Lichtstrahl.

In einer Ausgestaltung wird das Analysegerät dafür verwendet, um eine Gasprobe, die ein Proband abgegeben hat, auf eine vorgegebene Substanz zu untersuchen. Insbesondere vermag das Analysegerät eine vom Probanden ausgeatmete Gasprobe zu untersuchen. Die Substanz ist insbesondere Atemalkohol. Bei dieser Ausgestaltung umfasst das Analysegerät bevorzugt eine Eingabeeinheit, insbesondere ein Mundstück. Der Proband gibt die Atemprobe in die Eingabeeinheit ab, und die Messkammer steht über die einströmseitige Fluidverbindung mit der Eingabeeinheit in einer Fluidverbindung.

In einer Ausgestaltung ist das Analysegerät als ein tragbares Gerät ausgestaltet und umfasst eine eigene Spannungsversorgungseinheit. Dadurch lässt das Analysegerät sich beispielsweise dafür verwenden, um Fahrzeugführer an einem Kontrollpunkt zu kontrollieren. Nicht erforderlich ist, dass in diesem Kontrollpunkt ein Spannungsversorgungsnetz zur Verfügung steht. In vielen Fällen führt die Erfindung zu einem relativ geringen Verbrauch an elektrischer Energie, was insbesondere bei einer eigenen Spannungsversorgungseinheit wichtig ist. In einer anderen Ausgestaltung lässt das Analysegerät sich mit einem stationären Spannungsversorgungsnetz verbinden.

Im Folgenden wird die Erfindung anhand eines Ausführungsbeispiels beschrieben. Hierbei zeigt
- Figur 1: schematisch die Wirkungsweise eines elektrochemischen Sensors;
- Figur 2: den Probeneinlass eines erfindungsgemäßen Analysegeräts;
- Figur 3: in einer perspektivischen Darstellung schräg von oben ein erfindungsgemäßes Analysegerät gemäß einer ersten Ausgestaltung;
- Figur 4: in einer perspektivischen Darstellung schräg von oben den Antrieb und die angetriebenen Teile des Analysegeräts von Figur 3;
- Figur 5: in einer Darstellung von oben die in Figur 4 gezeigten Teile sowie die Lichtschranke und die Messkammer;
- Figur 6: ein senkrechter Schnitt durch das Analysegerät in der Ebene A - A von Figur 5;
- Figur 7: ein senkrechter Schnitt durch das Analysegerät in der Ebene B - B von Figur 5;
- Figur 8: in einer Detaildarstellung das Umsetzungs-Bauteil der ersten Ausgestaltung;
- Figur 9: die Abhängigkeit zwischen der Öffnungsweite des Ventils und der Rotationsposition der Nockenscheibe;
- Figur 10: in einer perspektivischen Darstellung schräg von oben ein erfindungsgemäßes Analysegerät gemäß einer zweiten Ausgestaltung;
- Figur 11: in einer perspektivischen Darstellung von oben den Antrieb, die Antriebsverbindung, die Messkammer und die Lichtschranke des Analysegeräts von Figur 10;
- Figur 12: in zwei Detaildarstellungen von vorne und von der Seite das Umsetzungs-Bauteil der zweiten Ausgestaltung;
- Figur 13: in einer perspektivischen Darstellung das Umsetzungs-Bauteil von Figur 12;
- Figur 14: in einer perspektivischen Darstellung das Umsetzungs-Bauteil der zweiten Ausgestaltung mit einer anderen Realisierungsform der Nockenscheibe;
- Figur 15: in einer Ansicht von der Seite das Umsetzungs-Bauteil von Figur 14.

Im Ausführungsbeispiel wird das erfindungsgemäße Analysegerät dafür verwendet, um eine Atemprobe, die ein Proband ausgeatmet hat, auf eine vorgegebene Substanz, insbesondere auf Atemalkohol, zu analysieren. Im Falle von Atemalkohol als der Substanz soll ein Proband daraufhin untersucht werden, ob sich in seinem Blut Alkohol oberhalb einer Nachweisgrenze befindet oder nicht. Der Proband gibt eine Atemprobe in ein Mundstück des Analysegeräts ein. Falls der Proband Alkohol zu sich genommen hat und der Alkohol im Blut noch nicht vollständig abgebaut ist, so enthält die abgegebene Atemprobe Atemalkohol. Ein Teil der abgegebenen Atemprobe fließt in eine Messkammer im Inneren des Analysegeräts. Dieser Teil fungiert als eine zu untersuchende Gasprobe und wird im Folgenden als "Messkammer-Probe" bezeichnet. Ein Sensor in oder an der Messkammer prüft, ob diese Messkammer-Probe Atemalkohol oder eine sonstige vorgegebene Substanz enthält oder nicht. Optional soll zusätzlich überprüft werden, ob die Luft im Mund des Probanden Atemalkohol enthält. Die Erfindung lässt sich auch für eine andere Substanz verwenden, die in der ausgeatmeten Luft eines Probanden oder in einem sonstigen Gas, das ein Proband abgeben kann, enthalten sein kann.

Der Sensor vermag ein Signal zu erzeugen, welches mit der Menge und / oder Konzentration der vorgegebenen Substanz in der Messkammer-Probe, welche in der Messkammer ist, korreliert. Aus dem Stand der Technik sind unterschiedliche geeignete Sensoren bekannt, beispielsweise elektrochemische Sensoren, photooptische Sensoren, photo-akustische Sensoren, Photo-Ionisierungs-Sensoren und Wärmetönungssensoren (katalytische Sensoren). Ein solcher Sensor lässt sich auch für die Erfindung anwenden.

Das Analysegerät leitet aus der Menge und / oder Konzentration von Atemalkohol in der Messkammer-Probe sowie der Menge und / oder dem Volumen der Messkammer-Probe die Konzentration von Atemalkohol in der eingegebenen Atemprobe ab. Die Menge und / oder das Volumen der Messkammer-Probe wird beispielsweise wie folgt hergeleitet:
- abhängig von dem Volumen der Messkammer und / oder
- abhängig von dem Volumen und / oder der Volumenänderung der unten beschriebenen Ansaug-Kammereinheit und / oder
- dadurch, dass der Volumenfluss in die Messkammer mehrfach gemessen wird und die Messwerte aufintegriert werden.

Das Volumen der Messkammer und das minimale und das maximale Volumen der Ansaug-Kammereinheit sind durch die Konstruktion des Analysegeräts bekannt.

Falls die Substanz Atemalkohol ist, so leitet eine signalverarbeitende Auswerteeinheit des Analysegeräts oder eine räumlich entfernte Auswerteeinheit aus der Atemalkohol-Konzentration in der Atemprobe den aktuellen Gehalt von Alkohol im Blut des Probanden ab.

Während die Atemprobe durch das Mundstück hindurch fließt, fließt zunächst ausgeatmete Luft aus dem Mund, dann ausgeatmete Luft aus den oberen Atemwegen und anschließend ausgeatmete Luft aus der Lunge des Probanden durch das Mundstück hindurch. Unter den "oberen Atemwegen" wird die Verbindung zwischen dem Mund und der Lunge des Probanden verstanden. Um festzustellen, ob der Proband gerade Alkohol getrunken hat, ist das Gas aus demjenigen Teil der Atemprobe zu untersuchen, die aus dem Mund des Probanden stammt, optional aus den oberen Atemwegen. Um festzustellen, ob das Blut des Probanden Alkohol enthält, ist ein Gas aus demjenigen Teil der Atemprobe zu untersuchen, der aus der Lunge stammt. Falls ausschließlich untersucht werden soll, ob das Blut des Probanden Alkohol enthält, so fließt idealerweise nur Gas aus der Lunge des Probanden in die Messkammer, und die Messkammer Probe enthält nur Luft aus der Lunge, aber keine Luft aus den oberen Atemwegen und keine Luft aus dem Mund. Falls zusätzlich untersucht werden soll, ob der Patient gerade Alkohol getrunken hat, so ist zusätzlich Gas aus dem Mund des Probanden zu untersuchen und muss ebenfalls in die Messkammer gelangen. In einer Ausgestaltung wird auch die Luft aus den oberen Atemwegen des Probanden untersucht. In einer anderen Ausgestaltung wird weitgehend verhindert, dass Luft aus den oberen Atemwegen in die Messkammer gelangt.

Das Analysegerät des Ausführungsbeispiels umfasst einen elektrochemischen Sensor 12. Unter einem "Sensor" wird ein Bauteil verstanden, welches automatisch ein Signal generiert, wobei das generierte Signal ein Maß für die Menge und / oder die Konzentration einer vorgegebenen Substanz in der Messkammer-Probe ist, wobei diese Messkammer-Probe sich in einer Messkammer befindet und wobei der Sensor diese Messkammer-Probe in der Messkammer zu analysieren vermag. Ein elektrochemischer Sensor löst eine chemische Reaktion aus, wobei die chemische Reaktion von der Menge und / oder Konzentration der zu analysierenden Substanz abhängt und eine messbare elektrische Detektions-Größe, beispielsweise die Stromstärke oder die elektrische Spannung oder die elektrische Ladung oder den elektrischen Widerstand eines Bauteils des Sensors, beeinflusst.

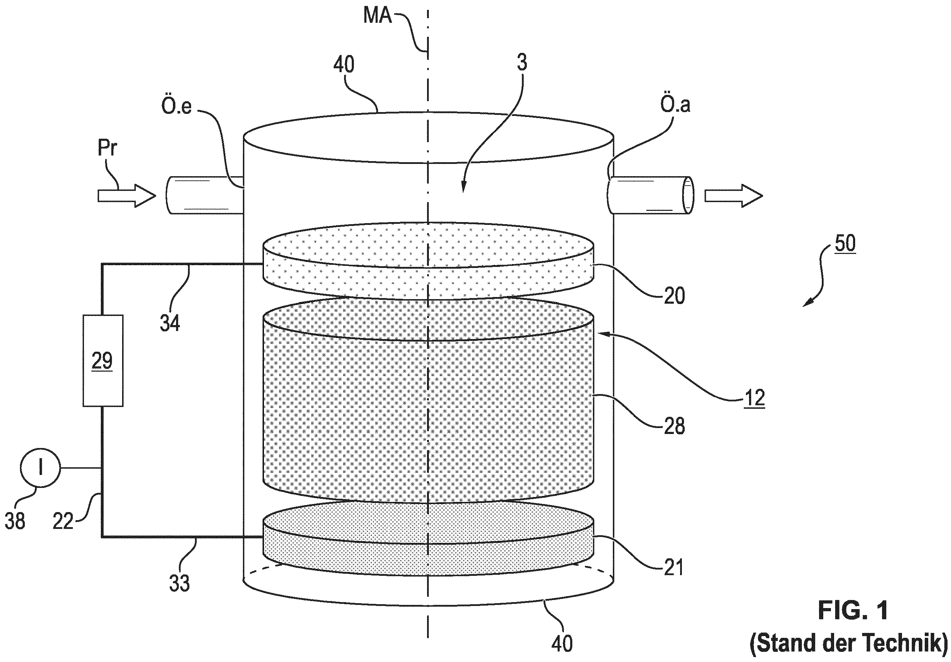

Figur 1 zeigt schematisch und beispielhaft die Wirkungsweise eines elektrochemischen Sensors 12, wie er aus dem Stand der Technik bekannt ist. Ein solcher Sensor 12 kann auch ein Bestandteil des erfindungsgemäßen Analysegeräts sein. Die Darstellung von Figur 1 ist nicht notwendigerweise maßstabsgerecht. Dieser elektrochemische Sensor 12 vermag eine Messkammer-Probe Pr auf Atemalkohol zu analysieren und arbeitet nach dem Prinzip einer Brennstoffzelle mit Alkohol als dem Brennstoff. Das erfindungsgemäße Analysegerät umfasst in einer Ausgestaltung einen derartigen elektrochemischen Sensor 12.

Mit dem Bezugszeichen 50 wird in Figur 1 eine Sensor-Anordnung bezeichnet, welche den eigentlichen elektrochemischen Sensor 12 und eine Wandung 40 für eine Messkammer 3 umfasst. Die Wandung 40 umgibt den Sensor 12 und die Messkammer 3. In der gezeigten Realisierungsform sind sowohl die Wandung 40 als auch der Sensor 12 rotationssymmetrisch zu derselben Mittelachse MA, die in der Zeichenebene liegt. Selbstverständlich sind auch andere geometrische Formen möglich.

Die zu analysierende Messkammer-Probe Pr, die im Ausführungsbeispiel von einer Atemprobe A stammt, fließt durch eine eintrittsseitige Öffnung Ö.e hindurch in das Innere der Messkammer 3, z.B. indem sie angesaugt wird und / oder in die Messkammer 3 diffundiert. In einer Ausgestaltung fließt die Messkammer-Probe Pr durch eine austrittsseitige Öffnung Ö.a wieder aus der Messkammer 3 heraus. Dank dieser Ausgestaltung kann der Sensor 12 rasch nacheinander mehrere Messkammer-Proben Pr untersuchen. Möglich ist auch, dass keine austrittsseitige Öffnung Ö.a vorhanden ist und die Messkammer-Probe Pr durch die eintrittsseitige Öffnung Ö.e hindurch wieder aus der Messkammer 3 herausfließt.

Der elektrochemische Sensor 12 umfasst
- eine Messelektrode 20, die durch einen Kontaktierungsdraht 34 elektrisch kontaktiert ist,
- eine Gegenelektrode 21, die durch einen Kontaktierungsdraht 33 elektrisch kontaktiert ist,
- einen Elektrolyten 28 zwischen den beiden Elektroden 20 und 21,
- einen Verbindungsdraht 22, der die beiden Kontaktierungsdrähte 33 und 34 miteinander elektrisch verbindet und einen elektrischen Messwiderstand 29 umfasst, und
- einen Stromstärken-Sensor 38, der die Stärke I des durch den Verbindungsdraht 22 fließenden Stroms misst.

Der Elektrolyt 28 wird bevorzugt von einer Membrane bereitgestellt. Ein solcher elektrochemischer Sensor 12 wird nachfolgend auch als Membran-Elektroden-Elektrolyt-Einheit (MPEE) bezeichnet.

Der Elektrolyt 28 ist ein elektrisch leitendes Medium, beispielsweise mit Wasser verdünnte Schwefelsäure oder Phosphorsäure oder Perchlorsäure. In dem Elektrolyten 28 können sich Ionen als elektrische Ladungsträger bewegen, was die elektrische Leitfähigkeit bewirkt. Bevorzugt stellt eine poröse Membrane den Elektrolyten 28 bereit. Der Elektrolyt 28 stellt eine ionisch leitfähige Verbindung zwischen der Messelektrode 20 und der Gegenelektrode 21 her.

Der Sensor 12 ist so ausgestaltet, dass die Messkammer-Probe Pr nur die Messelektrode 20 erreicht, aber nicht die Gegenelektrode 21. Im gezeigten Beispiel befindet die Messelektrode 20 sich an einer Wand der Messkammer 3, und die Wandung 40 und der Elektrolyt 28 verhindern, dass eine relevante Menge der Messkammer-Probe Pr die Gegenelektrode 21 erreicht.

Die beiden Kontaktierungsdrähte 33 und 34 sind elektrisch leitend und aus einem Material hergestellt, welches vom Elektrolyten 28 nicht chemisch angegriffen wird, beispielsweise aus Platin oder Gold. Auch die Elektroden 20 und 21 sind aus einem chemisch resistenten Material, beispielsweise ebenfalls aus Platin oder Gold. In vielen Fällen fungiert das chemisch resistente Material der Elektroden 20, 21 zusätzlich als Katalysator für eine chemische Reaktion, die zur Messung hervorgerufen und verwendet wird.

In einer Realisierungsform funktioniert der elektrochemische Sensor 12 nach dem Prinzip der Brennstoffzelle. Die chemische Reaktion, die zur Messung verwendet wird, umfasst den Schritt, dass in der Messkammer 3 Atemalkohol in der Messkammer-Probe Pr oxidiert wird. Idealerweise wird die gesamte Menge des Atemalkohols in der Messkammer-Probe Pr oxidiert.

Als Folge der chemischen Reaktion fließt ein elektrischer Strom zwischen der Messelektrode 20 und der Gegenelektrode 21 und damit durch den Verbindungsdraht 22 hindurch. Der Stromstärken-Sensor 38 misst ein Maß für die elektrische Ladung, also für die gesamte Menge des elektrischen Stroms, der durch den Verbindungsdraht 22 fließt (Prinzip der Coulometrie). In der Regel fließt elektrischer Strom so lange, bis alles elektrochemisch oxidierbare Gas, hier also Atemalkohol, in der Messkammer 3 tatsächlich elektrochemisch umgesetzt ist. Bei einem gegebenen Volumen der Messkammer-Probe Pr in der Messkammer 3 ist die gemessene elektrische Ladung Q umso höher, je mehr Atemalkohol die Messkammer-Probe Pr vor der elektrochemischen Umsetzung enthält. Die gemessene elektrische Ladung Q ist daher ein Maß für den Atemalkohol-Gehalt in der Messkammer-Probe Pr und somit für den Gehalt an Alkohol im Blut des Probanden.

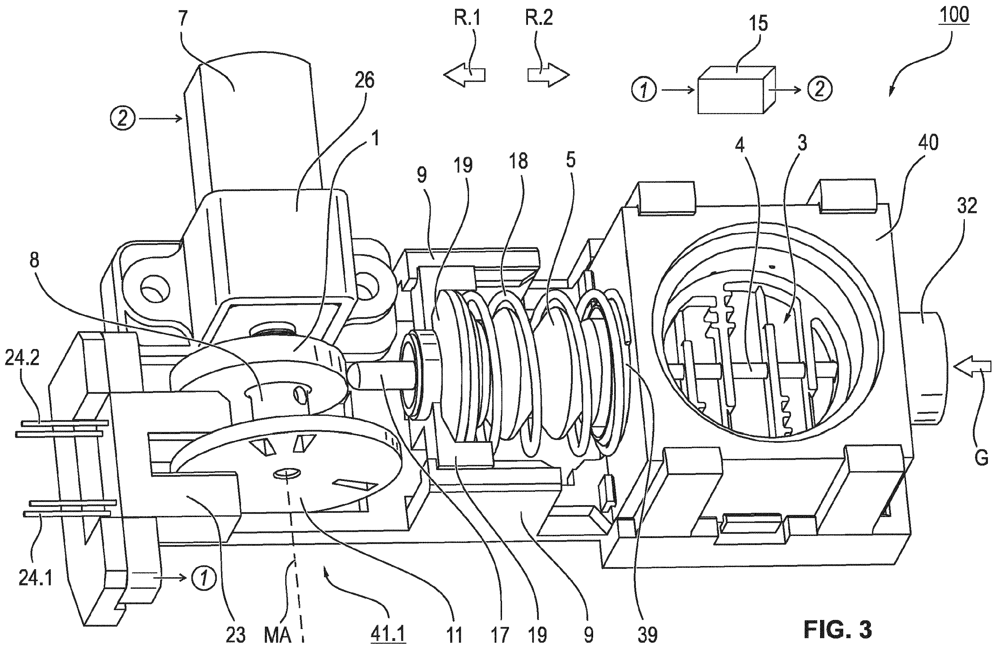

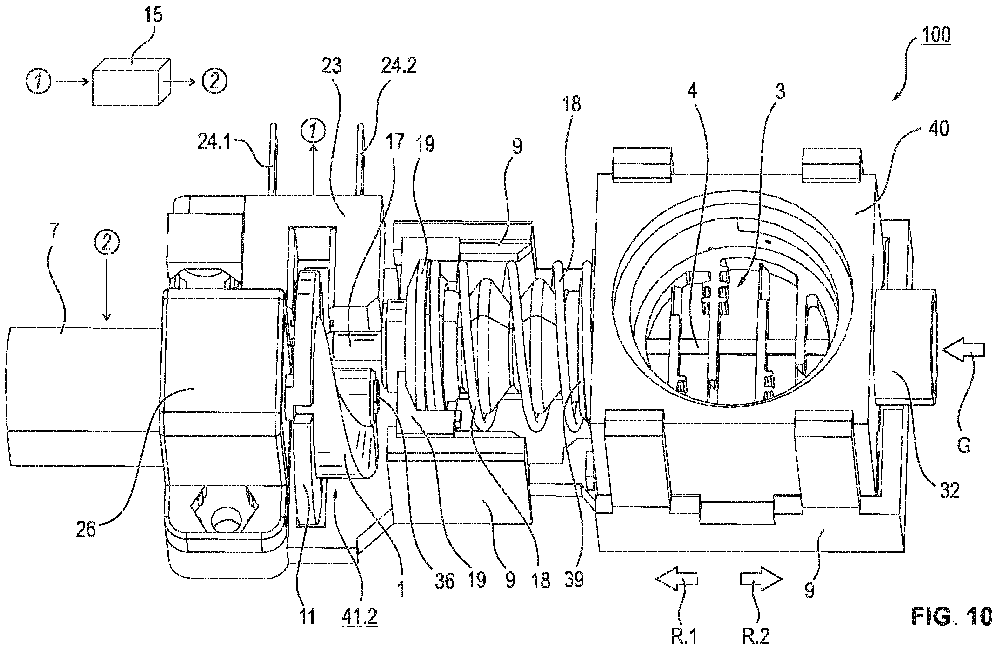

Figur 3 bis Figur 8 zeigen eine erste Ausgestaltung des erfindungsgemäßen Analysegeräts 100, Figur 10 bis Figur 15 eine zweite Ausgestaltung. Figur 1, Figur 2 und Figur 9 sind für beide Ausgestaltungen gültig.

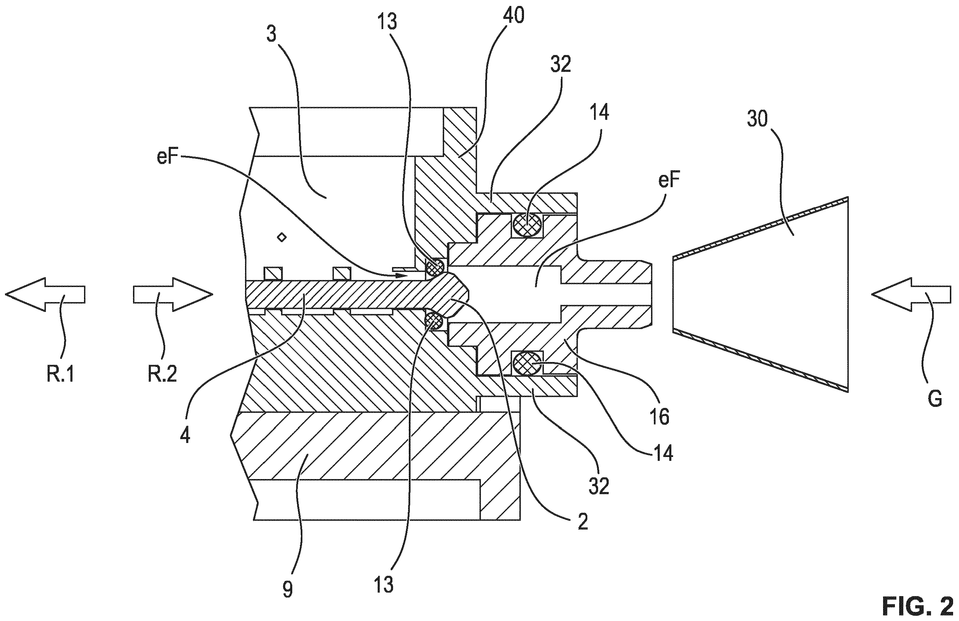

Figur 2 zeigt schematisch in einer Querschnittsdarstellung einen Probeneinlass 16 sowie eine Fluidverbindung zu der Messkammer 3. Auf den Probeneinlass 16 lässt sich ein trichterförmiges Mundstück 30 aufsetzen und wieder abnehmen. In einer Ausgestaltung detektiert ein nicht gezeigter Kontaktschalter das Ereignis, dass das Mundstück 30 auf den Probeneinlass 16 aufgesetzt ist. Die Wandung 40 umgibt die Messkammer 3 und den Sensor 12. Der Probeneinlass 16 ist mit der Wandung 40 verbunden und wird von einem einlassseitigen Anschlussstück 32 umgeben, welches fest oder auch lösbar mit der Wandung 40 verbunden ist. Ein Dichtring 14 sichert den Probeneinlass 16 im einlassseitigen Anschlussstück 32.

Ein Ventil mit einem beweglichen Ventilkörper 2 und einem Ventilkörper-Sitz in Form eines Dichtrings 13 vermag die Fluidverbindung zwischen dem Mundstück 30 und der Messkammer 3 wahlweise freizugeben oder zu verschließen. Diese Fluidverbindung wird im Folgenden als einströmseitige Fluidverbindung eF bezeichnet. Der Ventilkörper 2 lässt sich relativ zum Ventilkörper-Sitz 13 zwischen einer vollständig verschließenden und einer vollständig freigebenden (öffnenden) Endposition hin und her bewegen. In Figur 2 liegt der Ventilkörper 2 fluiddicht am Dichtring 13 an und verschließt die Fluidverbindung durch den Probeneinlass 16 hindurch, also die einströmseitige Fluidverbindung eF, vollständig. Indem der Ventilkörper 2 vom Dichtring 13 weg bewegt wird, und zwar in Figur 2 nach rechts, gibt das Ventil 2, 13 die einströmseitige Fluidverbindung eF frei, und Gas G kann aus dem Mundstück 30 durch die einströmseitige Fluidverbindung eF hindurch in die Messkammer 3 fließen.

Der Ventilkörper 2 ist fest auf ein zum Probeneinlass 16 zeigendes Ende einer Stange 4 montiert, in Figur 2 auf das rechte Ende. Die Stange 4 gehört zur Betätigungseinheit des Ausführungsbeispiels, erstreckt sich entlang einer Längsachse LA und vermag sich relativ zur Wandung 40 und relativ zum Dichtring 13 linear in zwei entgegengesetzte Richtungen R.1 und R.2 zu bewegen. Diese beiden Richtungen R.1, R.2 sind in Figur 2 horizontal dargestellt und liegen in der Zeichenebene. Bevorzugt wird die Stange 4 dergestalt geführt, dass sie sich ausschließlich in diese beiden Richtungen R.1 und R.2 bewegen kann. Eine Bewegung der Stange 4 in die erste Richtung R.1 verkleinert den Abstand zwischen dem Ventilkörper 2 und dem Dichtring 13, eine Bewegung in die zweite Richtung R.2 vergrößert diesen Abstand. Die beiden Richtungen R.1 und R.2 werden in Figur 2 durch zwei Pfeile angedeutet. Die Bewegung der Stange 4 bewirkt, dass das Ventil 2, 13 die Fluidverbindung eF wahlweise versperrt oder freigibt. In Figur 2 wird das Ventil 2, 13 in einer verschließenden Endposition gezeigt.

In der in Figur 2 gezeigten Realisierungsform befindet der Ventilkörper-Sitz 13 sich flussabwärts vom Ventilkörper 2. Das Ventil 2, 13 wird geöffnet, indem der Ventilkörper 2 in die zweite Richtung R.2 vom Dichtring 13 weg und auf das Mundstück 30 zu bewegt wird. Möglich ist auch die entgegengesetzte Realisierung, bei der sich der Ventilkörper-Sitz 13 flussaufwärts vom Ventilkörper 2 befindet und das Ventil 2, 13 dadurch geöffnet wird, dass der Ventilkörper 2 in die erste Richtung R.1 vom Mundstück 30 weg bewegt wird.

Figur 3 zeigt in einer perspektivischen Darstellung schräg von oben ein erfindungsgemäßes Analysegerät 100 gemäß der ersten Ausgestaltung. Eine Gasprobe G kann durch das Mundstück 30, den Probeneinlass 16 und das einströmseitige Anschlussstück 32 hindurch in die Messkammer 3 fließen, und zwar in Figur 3 horizontal von rechts nach links. An der quaderförmigen Wandung 40 der Messkammer 3 sind das einströmseitige Anschlussstück 32 sowie ein ausströmseitiges Anschlussstück 39 befestigt. In der Messkammer 3 ist ein Sensor angeordnet, der so wie in Figur 1 gezeigt aufgebaut sein kann und in Figur 3 fortgelassen ist.

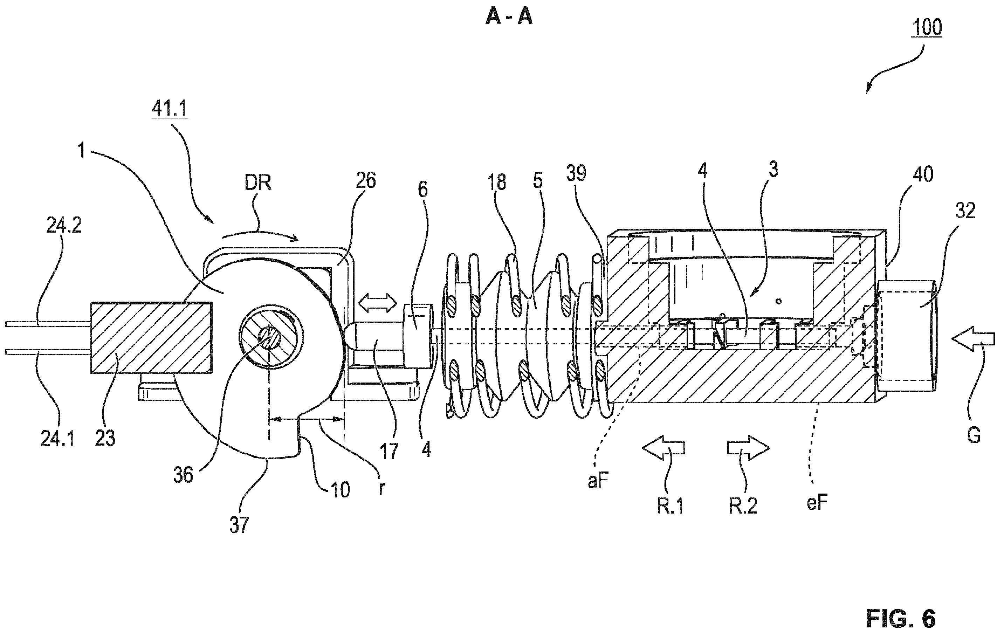

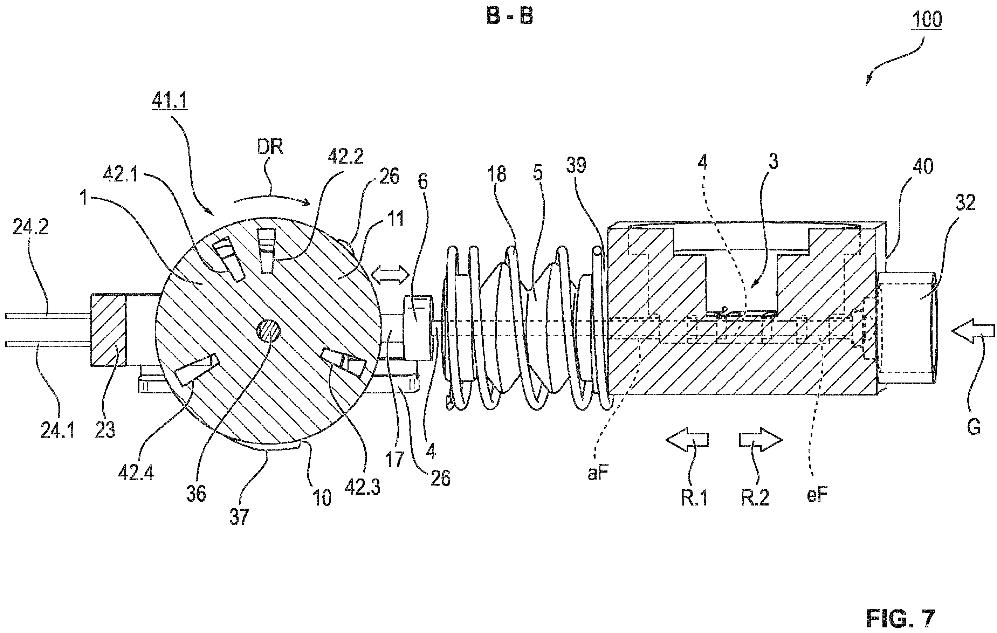

Die Messkammer 3 ist einerseits mit dem Mundstück 30 und damit mit der Umgebung mittels der einströmseitigen Fluidverbindung eF verbunden. Diese einströmseitige Fluidverbindung eF führt durch den Probeneinlass 16 und das einströmseitige Anschlussstück 32 hindurch. Andererseits steht die Messkammer 3 mit einem Balg 5 in einer ausströmseitigen Fluidverbindung aF. Diese ausströmseitige Fluidverbindung aF führt durch das ausströmseitige Anschlussstück 39 hindurch, vgl. Figur 6 und Figur 7.

Der Balg 5 gehört zur Ansaug-Kammereinheit des Ausführungsbeispiels, Ausführungsbeispiels, weist eine Wandung aus einem flexiblen Material auf und lässt sich wahlweise in einen Zustand mit minimalem Volumen und in einen Zustand mit maximalem Volumen überführen. In Figur 3 wird der Balg 5 in einem Zwischenzustand zwischen den beiden Endpositionen gezeigt. Der Schritt, den Balg 5 zu vergrößern, insbesondere in den Zustand mit maximalem Volumen zu überführen, bewirkt, dass Gas aus der Messkammer 3 durch die ausströmseitige Fluidverbindung aF hindurch in das Innere des Balg 5 angesaugt wird. Dadurch wird Gas G durch die einströmseitige Fluidverbindung eF hindurch in die Messkammer 3 gesaugt. Auf diese Weise gelangt auch ein Teil der Atemprobe, welche der Proband abgegeben hat, in die Messkammer 3. Der Schritt, den Balg 5 zu verkleinern, insbesondere in den Zustand mit minimalem Volumen zu überführen, bewirkt, dass Gas aus dem Balg 5 durch die ausströmseitige Fluidverbindung aF hindurch in die Messkammer 3 gefördert wird. Dadurch wird die Messkammer 3 gespült, und Gas fließt aus der Messkammer 3 durch die einströmseitige Fluidverbindung eF hindurch zurück in das Mundstück 30 und damit in die Umgebung.

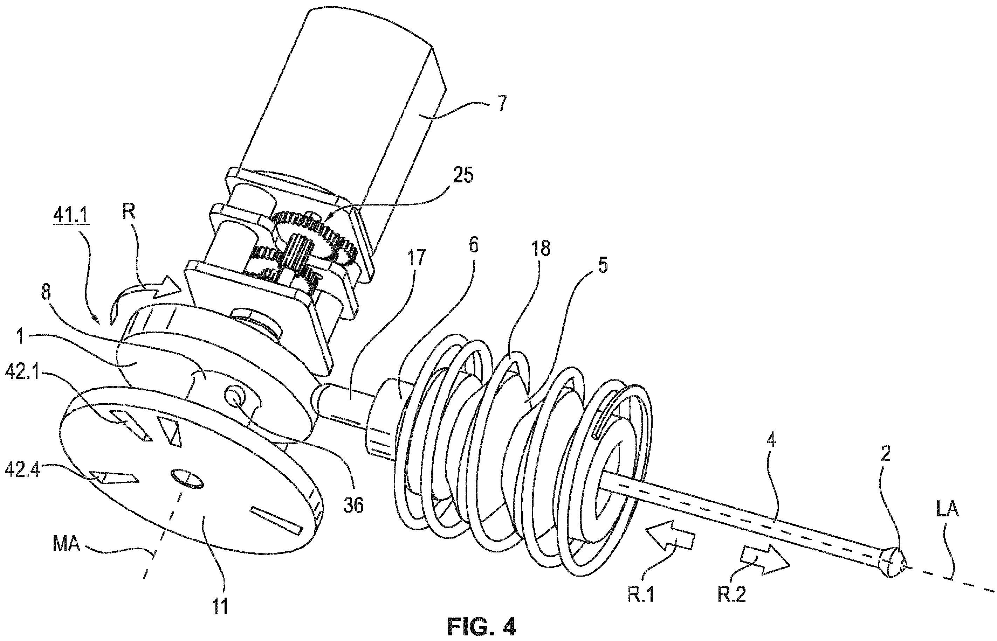

Figur 4 zeigt in einer perspektivischen Darstellung schräg von oben den Antrieb und die angetriebenen Teile des Analysegeräts 100 von Figur 3. Auf die Stange 4 ist eine Platte 6 dergestalt fest montiert, dass die Platte 6 senkrecht auf der Längsachse LA der Stange 4 steht und die Stange 4 durch die Platte 6 hindurch geführt ist. Diese Platte 6 überträgt eine Bewegung der Stange 4 auf den Balg 5. Falls die Stange 4 vom Mundstück 30 und vom Probeneinlass 16 weg bewegt wird, also im Beispiel von Figur 4 in die erste Richtung R.1 nach links, so bewirkt die Platte 6, dass der Balg 5 vergrößert, beispielsweise in den Zustand mit maximalem Volumen überführt wird. Dadurch wird Gas G in den Balg 5 gesaugt. Dadurch wiederum wird Gas G aus dem Mundstück 30 in die Messkammer 3 gesaugt. Am Ende der Bewegung ist der Ventilkörper 2 auf den Ventilkörper-Sitz 13 gezogen. Falls die Stange 4 in die entgegengesetzte Richtung R.2 bewegt wird, so wird der Balg 5 verkleinert, beispielsweise in den Zustand mit minimalem Volumen überführt. Dadurch wird Gas aus dem Balg 5 herausgefördert. Dadurch wiederum wird Gas aus der Messkammer 3 hinaus gefördert, und dadurch wird die Messkammer 3 gespült. Außerdem wird das Ventil 2, 13 geöffnet.

Um eine Gasprobe G in die Messkammer 3 zu saugen, wird bevorzugt folgende Abfolge durchgeführt:
- Anfänglich befindet sich der Balg 5 im Zustand mit maximalem Volumen, und das Ventil 2, 13 ist geschlossen. Solange das Ventil 2, 13 in der geschlossenen Endposition ist, wird verhindert, dass schädliche Gase und Partikel von außen durch die einströmseitige Fluidverbindung eF hindurch die Messkammer 3 erreichen. Außerdem wird verhindert, dass ein Bestandteil des Sensors 12, beispielsweise der Elektrolyt 28, verdunstet.
- Die Stange 4 wird in die zweite Richtung R.2 und damit auf den Probeneinlass 16 und das Mundstück 30 zu bewegt. Dieser Schritt wird durch ein erstes Ereignis ausgelöst, das im Folgenden als "spülungs-auslösendes Ereignis" bezeichnet wird. Durch die Bewegung der Stange 4 wird der Balg 5 in den Zustand mit minimalem Volumen überführt, und die Messkammer 3 wird gespült. Außerdem wird das Ventil 2, 13 geöffnet, sodass Gas aus der Messkammer 3 hinaus und durch die einströmseitige Fluidverbindung eF hindurch in das Mundstück 30 und damit in die Umgebung fließen kann.
- Die Stange 4 wird anschließend wieder in die erste Richtung R.1 und damit von dem Probeneinlass 16 und dem Mundstück 30 weg bewegt. Dieser Schritt wird durch ein zweites Ereignis ausgelöst, das im Folgenden als "ansaugauslösendes Ereignis" bezeichnet wird. Durch die Bewegung wird der Balg 5 wieder aus dem Zustand mit minimalem Volumen weg bewegt, also sein Volumen wird vergrößert. Dadurch wird eine Gasprobe durch die einströmseitige Fluidverbindung eF hindurch in die Messkammer 3 gesaugt. Außerdem wird das Ventil 2, 13 aus der vollständig freigebenden Endposition weg bewegt, bevorzugt in eine Zwischenposition.
- Zum Abschluss wird wieder der anfängliche Zustand hergestellt, d.h. die Stange 4 wird weiter in die erste Richtung R.1 bewegt, und dadurch werden das Ventil 2, 13 vollständig geschlossen und der Balg 5 in den Zustand mit maximalem Volumen überführt.

Die Stange 4 bewirkt im Ausführungsbeispiel folgende Synchronisation von zwei Vorgängen: Der Vorgang, den Balg 5 zu vergrößern oder zu verkleinern, ist mit dem Vorgang synchronisiert, den Ventilkörper 2 relativ zum Ventilkörper-Sitz 13 zu bewegen. Dank der Stange 4 ist das Ventil 2, 13 dann vollständig geschlossen, wenn der Balg 5 den Zustand mit maximalem Volumen aufweist. Das Ventil 2, 13 ist vollständig geöffnet, wenn der Balg 5 den Zustand mit minimalem Volumen aufweist. Möglich ist auch eine umgekehrte Synchronisation, also dass dank der Stange 4 das Ventil 2, 13 dann geschlossen ist, wenn der Balg 5 den Zustand mit minimalem Volumen aufweist.

Wie gerade beschrieben, wird der Balg 5 vergrößert, beispielsweise in den Zustand mit maximalem Volumen überführt, und dadurch wird eine Gasprobe G in die Messkammer 3 gesaugt. Anschließend wird der Balg 5 wieder verkleinert, beispielsweise in den Zustand mit minimalem Volumen überführt. Dadurch wird die Messkammer 3 gespült.

Bevorzugt wird der Balg 5 durch eine rasche Bewegung aus dem Zustand mit minimalem Volumen heraus vergrößert, wodurch eine Gasprobe G in die Messkammer 3 gesaugt wird. "Rasche Bewegung" bedeutet: Diese Bewegung dauert höchstens eine Sekunde, bevorzugt weniger als 100 Millisekunden. Dadurch wird eine definierte Menge des Gases in die Messkammer 3 gesaugt, und nur wenig Gas diffundiert in die Messkammer 3.

Möglich ist, dass der Balg 5 durch eine genauso rasche Bewegung wieder verkleinert und in den Zustand mit minimalem Volumen überführt wird.

In einer bevorzugten Ausgestaltung wird der Balg 5 hingegen durch eine wesentlich langsamere Bewegung wieder in den Zustand mit minimalem Volumen überführt, wobei diese langsamere Bewegung bevorzugt mindestens eine halbe Sekunde, besonders bevorzugt mindestens eine Sekunde, insbesondere zehn Sekunden dauert. Bei dieser Bewegung kann sich die Geschwindigkeit ändern, mit der die Stange 4 in die zweite Richtung R.2 auf den Probeneinlass 16 zu bewegt wird.

Bevorzugt verschließt das Ventil 2, 13 erst dann vollständig die einströmseitige Fluidverbindung eF, wenn ein zweites verschließendes Ereignis eingetreten ist. Solange die einströmseitige Fluidverbindung eF noch nicht vollständig geschlossen ist, findet ein Druckausgleich zwischen dem Druck in der Messkammer 3 und dem Umgebungsdruck statt. Anmerkung: Aufgrund eines pneumatischen Fließwiderstands in der einströmseitigen Fluidverbindung eF benötigt dieser Druckausgleich häufig einige Zeit. Außerdem vermeidet der Druckausgleich das unerwünschte Ereignis, dass das Messergebnis des Sensors 12 davon beeinflusst wird, wie dicht das Ventil 2, 13 tatsächlich die einströmseitige Fluidverbindung eF verschließt.

In dem Ausführungsbeispiel, das mit Bezug auf die Figuren beschrieben wird, wird von jedem Probanden jeweils eine einzige Gasprobe G in die Messkammer 3 geleitet. Idealerweise besteht diese Gasprobe G ausschließlich aus Luft, welche aus der Lunge des Probanden stammt, aber weder aus Luft aus dem Mund noch aus Luft aus den oberen Atemwegen des Probanden. Durch das öffnende Ereignis lässt sich relativ gut festlegen, welcher Teil der Atemprobe in die Messkammer 3 gesaugt wird.

Das spülungs-auslösende Ereignis bewirkt, dass die Messkammer 3 gespült wird. Dieses Ereignis ist beispielsweise dann eingetreten, wenn der oben erwähnte Kontaktschalter das Ereignis detektiert, dass das Mundstück 30 auf den Probeneinlass 16 aufgesetzt ist. Weil die Messkammer 3 gespült wird und Gas durch die einströmseitige Fluidverbindung eF hindurch aus der Messkammer 3 strömt, wird verhindert, dass Gas aus dem Mundstück 30 durch den Probeneinlass 16 hindurch in die Messkammer 3 strömt.

Das ansaug-auslösende Ereignis löst den Schritt aus, dass die Stange 4 in die erste Richtung R.1 von dem Probeneinlass 16 weg bewegt wird und dadurch den Balg 5 aus dem Zustand mit minimalem Volumen weg bewegt und dadurch das Volumen des Balgs 5 vergrößert. In einer Ausgestaltung ist das ansaug-auslösende Ereignis eingetreten, wenn seit dem Vorgang, das Mundstück 30 auf den Probeneinlass 16 aufzusetzen, eine vorgegebene Zeitspanne verstrichen ist. In einer anderen Ausgestaltung misst ein nicht gezeigter Volumenfluss-Sensor ein Maß für den Volumenfluss in das Mundstück 30 und leitet hieraus das Volumen der Menge von Gas ab, welches bislang in das Mundstück 30 oder durch das Mundstück 30 hindurch in die Messkammer 3 geflossen ist. Wenn dieses Volumen eine vorgegebene Volumenschranke erreicht hat, so ist alle Luft aus dem Mund und den oberen Atemwegen des Probanden in das Mundstück 30 geflossen, und es folgt Luft aus der Lunge des Probanden. Die Volumenschranke wird beispielsweise so vorgegeben, dass sie gleich dem durchschnittlichen Volumen der oberen Atemwege und des Mundes eines erwachsenen Probanden ist. Das ansaug-auslösende Ereignis ist eingetreten, wenn das Volumen, das bislang in das Mundstück 30 geflossen ist, die Volumenschranke erreicht hat.

In einer bevorzugten Ausgestaltung wird der Balg 5 durch eine rasche Bewegung in die erste Richtung R.1 aus dem Zustand mit minimalem Volumen heraus bewegt und vergrößert, wodurch eine Gasprobe in die Messkammer 3 gesaugt wird, ohne sofort in den Zustand mit maximalem Volumen überführt zu werden. Die rasche Bewegung dauert bevorzugt weniger als 1 sec, besonders bevorzugt weniger als 100 msec. Das Ventil 2, 13 wird durch die rasche Bewegung aus der vollständig freigebenden Endposition in eine Zwischenposition überführt. Durch eine nachfolgende langsamere Bewegung in die erste Richtung R.1 wird der Balg 5 in den Zustand mit maximalem Volumen überführt. Das Ventil 2, 13 wird vollständig geschlossen. Solange, bis das Ventil 2, 13 vollständig geschlossen ist, bleibt die einströmseitige Fluidverbindung eF geöffnet. Daher kann der oben beschriebene Druckausgleich zwischen der Messkammer 3 und der Umgebung stattfinden. Auch während der nachfolgenden langsameren Bewegung wird Gas in die Messkammer 3 gesaugt, aber deutlich weniger als bei der raschen Bewegung.

Bevorzugt wird das Ventil 2, 13 dann durch eine Bewegung in die erste Richtung R.1 in die vollständig verschließende Endposition bewegt, wenn ein vorgegebenes verschließendes Ereignis eingetreten ist. In einer Realisierungsform ist das verschließende Ereignis dann eingetreten, wenn die chemische Reaktion des Sensors 12 vollständig abgeschlossen ist. In einer Ausgestaltung wird dieses Ereignis dadurch festgestellt, dass die Stromstärke des Stroms, der durch die Leitung 22 des Sensors 12 von Figur 1 fließt, unter eine vorgegebene Schranke fällt. Dies bedeutet, dass der elektrochemische Sensor 12 allen Atemalkohol in der Messkammer 3 umgesetzt hat.

Durch eine nachfolgende weitere langsame Bewegung in die zweite Richtung R.2 wird der Balg 5 aus dem Zustand mit maximalem Volumen wieder in den Zustand mit minimalem Volumen überführt. Das Ventil 2, 13 wird wieder geöffnet. Die Messkammer 3 wird gespült.

Wie bereits dargelegt, lässt sich die Stange 4 in zwei einander entgegengesetzte Richtungen R.1 und R.2 linear bewegen. Dies wird in Figur 2, Figur 3 und Figur 4 durch zwei einander entgegengesetzte Pfeile R.1 und R.2 angedeutet. Die Bewegung der Stange 4 überführt sowohl den Balg 5 von dem einen Zustand in den anderen Zustand als auch den Ventilkörper 2 relativ zum Ventilkörper-Sitz 13 von der einen Endposition in die andere Endposition. Im Folgenden wird eine bevorzugte Ausgestaltung dargelegt, wie diese Stange 4 bewegt wird.

Auf dasjenige freie Ende der Stange 4, die von dem Ventil 2, 13 abgewandt ist, ist ein Kontaktelement in Form eines Stößels 17 montiert. Die Stange 4 ist durch eine runde Öffnung in einem Verbindungselement 19 hindurch geführt, vgl. Figur 3. Der Durchmesser des Stößels 17 ist größer als der Durchmesser dieser runden Öffnung im Verbindungselement 19. Eine Druckfeder 18 stützt sich an der Wandung 40 ab und ist bestrebt, das Verbindungselement 19 und damit den Stößel 17 und die Stange 4 weg von dem Probeneinlass 16 und weg von dem einströmseitigen Anschlussstück 32 zu bewegen, also in die erste Richtung R.1. Der Stößel 17 und damit die Stange 4 lassen sich gegen die Federkraft der Druckfeder 18 auf das einströmseitige Anschlussstück 32 zu bewegen, also in die zweite Richtung R.2. In Figur 4 sind das Verbindungselement 19, das einströmseitige Anschlussstück 32 und die Wandung 40 fortgelassen.

Ein ansteuerbarer Elektromotor 7 vermag eine Ausgangswelle 36 um die Mittelachse MA der Ausgangswelle 36 zu drehen. Ein Untersetzungsgetriebe 25 verbindet den Elektromotor 7 mit der Ausgangswelle 36. Weil der Elektromotor 7 ausschließlich eine Drehbewegung ausführt und über das Untersetzungsgetriebe 25 mit der Ausgangswelle 36 verbunden ist, vermag der Elektromotor 7 ein höheres Drehmoment zu erzeugen als ein anderer Motor mit gleichem Bauraum. Außerdem braucht der Elektromotor 7 keine oszillierende Bewegung auszuführen, sondern nur eine rotierende Bewegung stets in eine Drehrichtung. Das Untersetzungsgetriebe 25 ist durch eine Abdeckung 26 vor Schmutzpartikeln und sonstigen Umgebungseinflüssen geschützt. Die Abdeckung 26 ist in Figur 4 fortgelassen.

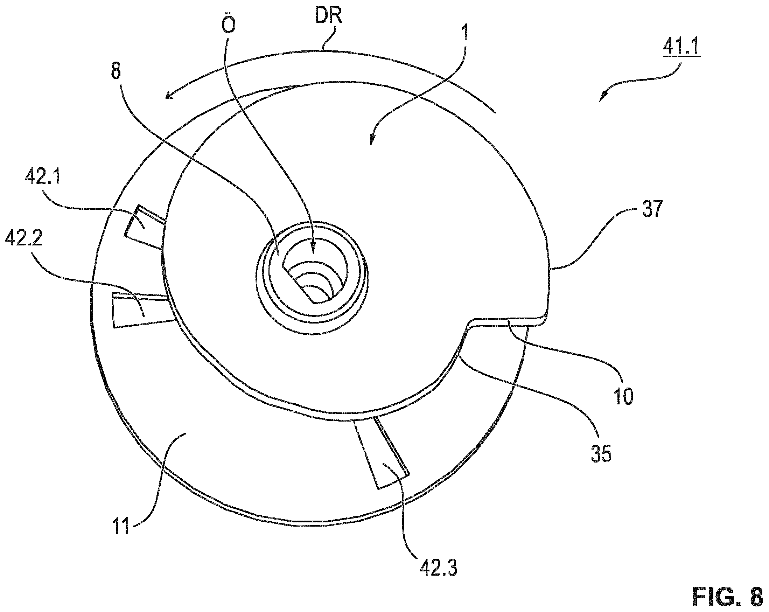

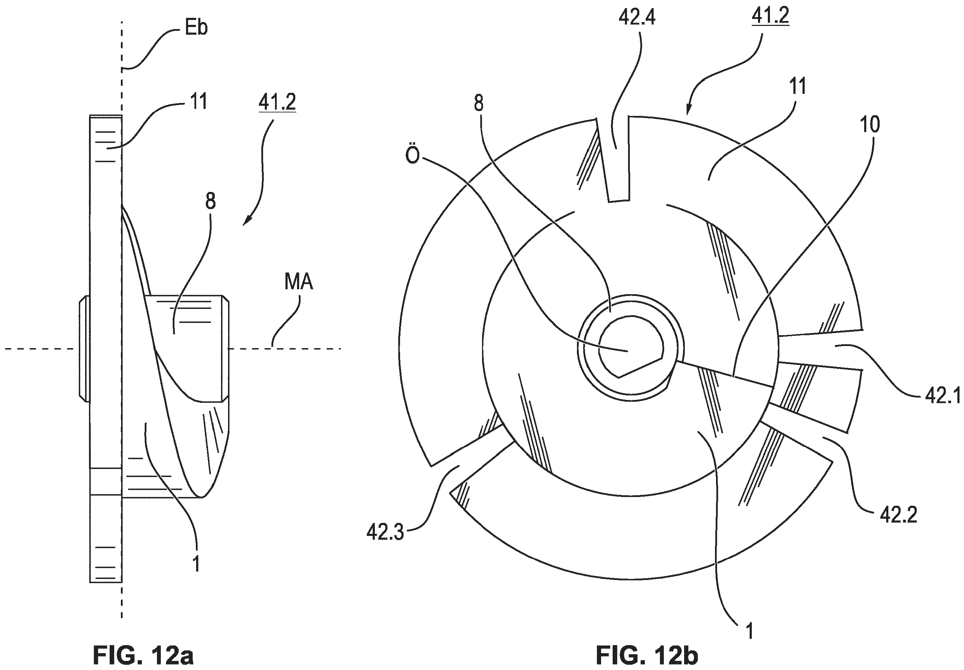

Die Ausgangswelle 36 ist durch ein drehbar gelagertes Umsetzungs-Bauteil 41.1 hindurchgeführt und drehfest mit dem Umsetzungs-Bauteil 41.1 verbunden. Die Drehachse und Mittelachse MA der Ausgangswelle 36 ist zugleich die Drehachse des Umsetzungs-Bauteils 41.1. Das Umsetzungs-Bauteil 41.1 umfasst eine hohle Nockenwelle 8, eine Nockenscheibe 1 und eine kreisrunde Lochscheibe (Encoder-Scheibe) 11. Die Ausgangswelle 36 ist durch eine Öffnung Ö in der Nockenwelle 8 hindurchgeführt. Die Kontur der Öffnung Ö bewirkt eine formschlüssige Verbindung zwischen der Ausgangswelle 36 und dem Umsetzungs-Bauteil 41.1, vgl. Figur 8 und Figur 12.

Die Nockenscheibe 1 und die Lochscheibe 11 erstrecken sich in zwei zueinander parallelen Ebenen, die beide senkrecht auf der Mittelachse MA stehen. Die Stange 4 liegt in derjenigen Ebene, in der sich die Nockenscheibe 1 erstreckt. Die Längsachse LA der Stange 4 steht senkrecht auf der Mittelachse MA. Die Druckfeder 18 ist bestrebt, den Stößel 17 gegen die Umfangskontur der Nockenscheibe 1 zu drücken und in Kontakt mit dieser Umfangskontur zu halten, und zwar auch dann, wenn die Nockenscheibe 1 gedreht wird.

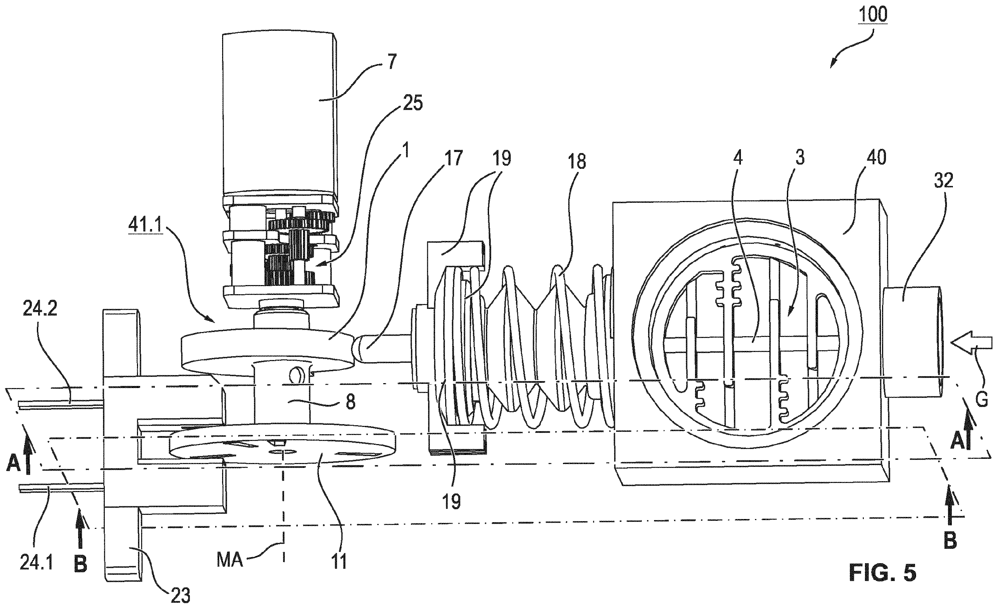

Figur 5 zeigt in einer perspektivischen Darstellung von oben das Analysegerät 100. Das Gestell 9 und die Abdeckung 26 sind fortgelassen. Figur 6 zeigt das Analysegerät 100 in der Ebene A - A von Figur 5, Figur 7 in der Ebene B - B. Die Mittelachse MA liegt in der Zeichenebene von Figur 5 und steht senkrecht auf den Zeichenebenen von Figur 6 und Figur 7. Die Längsachse LA liegt in den Zeichenebenen von Figur 5, Figur 6 und Figur 7. Figur 8 zeigt in einer perspektivischen Darstellung das Umsetzungs-Bauteil 41.1 gemäß der ersten Ausgestaltung, wobei die Nockenscheibe 1 zum Betrachter hin zeigt.

Die Ausgangswelle 36 dreht die Nockenscheibe 1 in die Drehrichtung DR um die Mittelachse MA, und zwar im Beispiel von Figur 6 und Figur 7 in die Uhrzeigerrichtung. Bei dieser Bewegung nimmt der Radius r der Nockenscheibe 1 von einem minimalen Radius rₘᵢₙ allmählich zu, bis die Nockenscheibe 1 ihren maximalen Radius rₘₐₓ erreicht. Unter dem Radius r wird der Abstand zwischen der Umfangskontur und der Mittelachse MA gemessen an der Stelle, an welcher der Stößel 17 die Umfangskontur berührt, verstanden. In einem Segment 37 weist die Umfangskontur der Nockenscheibe 1 durchgehend diesen maximalen Radius rₘₐₓ auf. Die Umfangskontur umfasst außerdem eine Kante 10, in der sich der Radius r schlagartig ändert, und zwar bei der Drehung in die Drehrichtung DR im Uhrzeigersinn schlagartig verkleinert.

Jede Rotationsposition der Nockenscheibe 1 legt eine Position des Ventilkörpers 2 relativ zum Ventilkörper-Sitz 13 fest. Außerdem legt jede Rotationsposition eine Position der Platte 6 und damit ein Volumen des Balgs 5 fest. Diese beiden Effekte werden erzielt, weil dank der Druckfeder 18 der Stößel 17 und damit die Stange 4 der Umfangskontur der Nockenscheibe 1 folgt und weil sowohl die Platte 6 als auch der Ventilkörper 2 mit der Stange 4 fest verbunden sind.

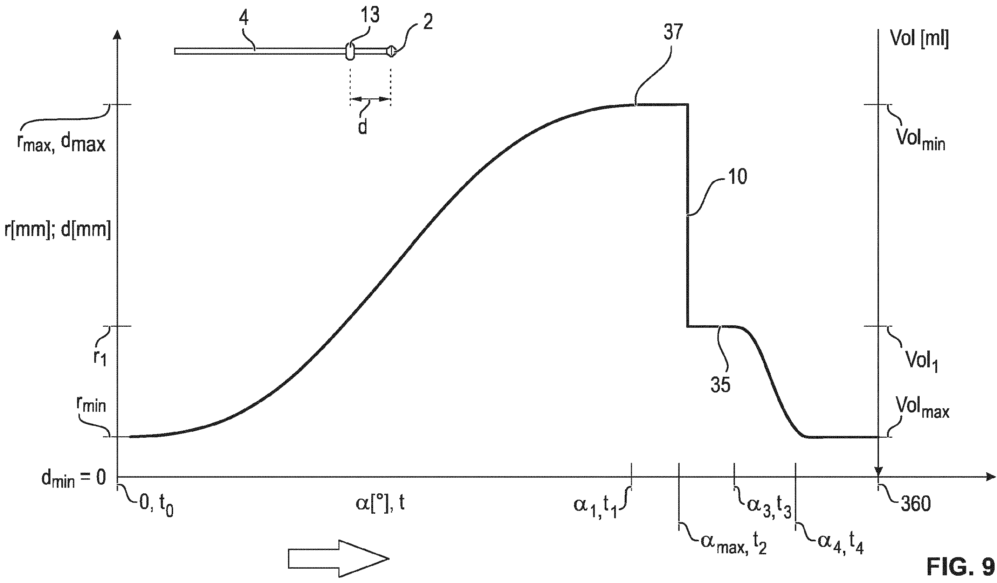

Figur 9 zeigt beispielhaft diese Abhängigkeit. Figur 9 ist eine schematische und nicht notwendigerweise maßstabsgerechte Darstellung. Auf der x-Achse ist die Rotationsposition α der Nockenscheibe 1 relativ zum Stößel 17 gezeigt. Dargestellt ist eine volle Drehung der Nockenscheibe 1, wobei die Rotationsposition α=0°=360° eine vorgegebene Referenz-Rotationsposition ist, bei welcher der Stößel 17 den kleinstmöglichen Abstand zur Nockenwelle 8 aufweist. Auf der linken y-Achse sind der jeweils festgelegte Radius r der Nockenscheibe 1 bei der jeweiligen Rotationsposition α sowie die daraus resultierende Position des Ventilkörpers 2 relativ zum Ventilkörper-Sitz 13 gezeigt, wobei die Ventilkörper-Position als Abstand d und damit als Öffnungsweite des Ventils 2, 13 dargestellt wird. Dieser Abstand d wird in Figur 9 oben veranschaulicht. Auf der rechten y-Achse ist das Volumen Vol des Balgs 5, das durch die jeweilige Rotationsposition α festgelegt ist, angedeutet. Die Rotationspositionen sind so gewählt, dass die Rotationsposition von 0° bis 360° ansteigt, weswegen die x-Achse auch für die fortlaufende Zeit t steht. In Figur 9 ist eingetragen, welchen Verlauf die Kante 10 und die beiden Segmente 35 und 37 bewirken.

Die Rotationsposition α=0° der Nockenscheibe 1 legt folgendes fest:
- den kleinstmöglichen Abstand dₘᵢₙ=0 des Ventilkörpers 2 vom Ventilkörper-Sitz 13, also die vollständig verschließende Endposition,
- den minimalen Radius rₘᵢₙ der Nockenscheibe 1 und
- das maximale Volumen Volₘₐₓ des Balgs 5.

Die Kante 10 ist an einer Rotationsposition α=αₘₐₓ positioniert. Die Rotationsposition α=αₘₐₓ legt folgendes fest:
- den maximalen Abstand dₘₐₓ des Ventilkörpers 2 vom Ventilkörper-Sitz 13, also die vollständig öffnende Endposition,
- den maximalen Radius rₘₐₓ der Nockenscheibe 1 und
- das minimale Volumen Volₘᵢₙ des Balgs 5.

Anfänglich befindet die Nockenscheibe 1 sich in der Rotationsposition α=0°. Zum Zeitpunkt t₀ wird das spülungs-auslösende Ereignis detektiert. Der Motor 7 wird aktiviert. Die Nockenscheibe 1 wird gedreht, bis sie in der Rotationsposition α₁ und zum Zeitpunkt t₁ ihren maximalen Radius rₘₐₓ annimmt und der Stößel 17 das Segment 37 erreicht. Bei einer weiteren Drehung gleitet der Stößel 17 über das Segment 37, und der Radius r bleibt im maximalen Wert rₘₐₓ. Der Balg 5 ist im Zustand mit minimalem Volumen Volₘᵢₙ. Die Nockenscheibe 1 wird bevorzugt gestoppt.

Zum Zeitpunkt t₂ wird das ansaug-auslösende Ereignis detektiert. Der Stößel 17 gleitet über die Kante 10. Der Radius r verringert sich schlagartig von rₘₐₓ auf einen kleineren Wert r₁, der aber noch größer als der minimale Radius rₘᵢₙ ist. Die Druckfeder 18 dehnt sich aus und bewirkt, dass das Volumen des Balgs 5 sich auf den Wert Vol₁ vergrößert. Dadurch wird eine Gasprobe G durch die einströmseitige Fluidverbindung eF hindurch in die Messkammer 3 gesaugt. Die Kante 10 kann radial oder sogar etwas schräg angeordnet sein, sodass der Stößel 17 zeitweise den Kontakt zur Nockenscheibe 1 verliert. Dies reduziert die Reibung zwischen dem Stößel 17 und der Nockenscheibe 1.

Bei einer weiteren Drehung der Nockenscheibe 1 nimmt der Radius r allmählich ab, und das Volumen Vol des Balgs 5 nimmt zu, bis wieder der minimale Radius rₘᵢₙ und das maximale Volumen Volₘₐₓ wieder erreicht sind. Im Beispiel von Figur 9 bleibt der Radius r im Zeitraum von t₂ bis t₃ konstant, nämlich mit dem Wert r₁, und nimmt dann ab, bis er zum Zeitpunkt t₄ wieder minimal geworden ist. Dass der Radius r im Zeitraum von t₂ bis t₃ auch bei einer Rotation der Nockenscheibe 1 den gleichbleibenden Wert r₁ beibehält, wird durch ein Segment 35 erzielt, vgl. Figur 8.

Bevorzugt vermag ein signalverarbeitendes Steuergerät (control unit) 15 Messwerte von verschiedenen Sensoren des Analysegeräts 100 zu empfangen und zu verarbeiten und den Elektromotor 7 zu aktivieren und zu deaktivieren.

Dadurch wird der Elektromotor 7 in einem Start-Stopp-Betrieb betrieben, und zwar abhängig von mindestens einem der oben beschriebenen Ereignisse.

Anfänglich ist der Elektromotor 7 deaktiviert, und die Nockenscheibe 1 wird nicht gedreht. Das spülungs-auslösende Ereignis bewirkt, dass das Steuergerät 15 den Elektromotor 7 aktiviert und der aktivierte Elektromotor 7 die Ausgangswelle 36 und damit das Umsetzungs-Bauteil 41.1 mit der Nockenscheibe 1 dreht.

In einer Ausgestaltung bleibt der Elektromotor 7 so lange aktiviert, bis die vom Elektromotor 7 gedrehte Nockenscheibe 1 eine vollständige Umdrehung vollführt hat. Die Geometrie der Umfangskontur der Nockenscheibe 1 sowie die Drehgeschwindigkeit legen fest, wann das ansaug-auslösende Ereignis und wann das verschließende Ereignis eingetreten sind.

In einer bevorzugten Ausgestaltung deaktiviert das Steuergerät 15 hingegen mehrmals den Elektromotor 7 und aktiviert ihn wieder, während der Elektromotor 7 die Nockenscheibe 1 dreht. Dadurch wird ein ereignisgesteuerter Betrieb realisiert. Dieser wird nachfolgend beschrieben.

Der Elektromotor 7 wird wieder deaktiviert, und die Drehung der Nockenscheibe 1 wird unterbrochen, wenn die Rotationsposition zwischen den Werten α₁ und αₘₐₓ liegt, bevorzugt nahe dem Wert αₘₐₓ. Zum Zeitpunkt t₂ wird das ansaug-auslösende Ereignis detektiert, und der Elektromotor 7 wird wieder aktiviert und dreht die Nockenscheibe 1. Dadurch und dank der Kante 10 wird das Volumen Vol des Balgs 5 schlagartig vom minimalen Volumen Volₘᵢₙ auf den Wert Vol₁ vergrößert. Der Elektromotor 7 dreht die Nockenscheibe 1 weiter, beispielsweise bis zu Rotationsposition α₃, und wird dann wieder deaktiviert. Dank des Segments 35 bleiben der Radius r und das Volumen Vol auch bei sich drehender Nockenscheibe 1 auf dem gleichen Werten r₁ bzw. Vol₁. Zum Zeitpunkt t₃ wird das Ereignis detektiert, dass die chemische Reaktion im Sensor 12 abgeschlossen ist. Diese Detektion fungiert als das verschließende Ereignis. Der Elektromotor 7 wird wieder aktiviert und dreht die Nockenscheibe 1 bis zu Rotationsposition α=0°. Der Radius r beträgt nunmehr wieder rₘᵢₙ, und das Volumen Vol beträgt Volₘₐₓ.

Die Betriebssicherheit beim ereignisgesteuerten Betrieb wird erhöht, wenn ein Sensor die aktuelle Rotationsposition der Nockenscheibe 1 misst. Im Ausführungsbeispiel ist auf der Nockenwelle 8 zusätzlich zu der Nockenscheibe 1 eine Lochscheibe (Encoder-Scheibe) 11 drehfest montiert. Die Nockenwelle 8, die Nockenscheibe 1 und die Lochscheibe 11 bilden im Ausführungsbeispiel ein einziges Bauteil, nämlich das Umsetzungs-Bauteil 41.1. In die Lochscheibe 11 ist mindestens eine Aussparung eingelassen, bevorzugt mehrere Aussparungen 42.1, 42.2, .... Die Lochscheibe 11 unterbricht den Lichtstrahl einer Lichtschranke 23. Die Aussparungen 42.1, 42.2, .... lassen diesen Lichtstrahl passieren. Zwei elektrische Kontaktierungen 24.1 und 24.2 versorgen die Lichtschranke 23 mit elektrischer Energie. Das Steuergerät 15 empfängt ein Signal von diesem Positionssensor umfassend die Lichtschranke 23 und die Lochscheibe 11 und leitet die aktuelle Rotationsposition α der Nockenscheibe 1 ab.

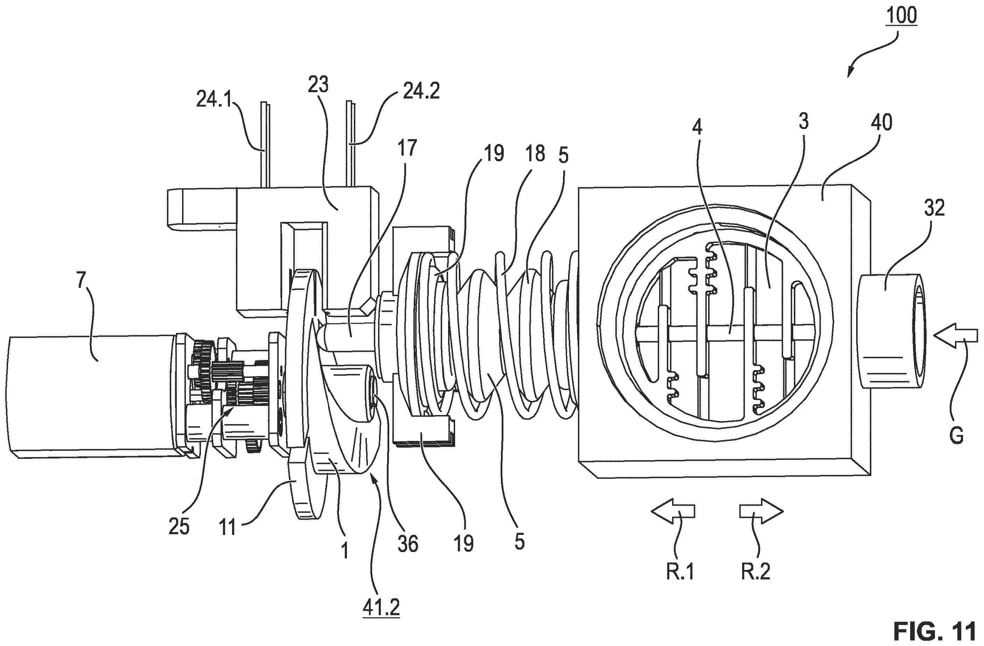

Figur 10 bis Figur 15 zeigen eine zweite Ausgestaltung des erfindungsgemäßen Analysegeräts 100. Gleiche Bezugszeichen haben die gleiche Bedeutung wie in den Figuren, die sich auf die erste Ausgestaltung beziehen. Figur 10 zeigt die zweite Ausgestaltung in einer perspektivischen Darstellung schräg von oben. Figur 11 zeigt die zweite Ausgestaltung in einer perspektivischen Darstellung von oben, wobei das Gestell 9 und die Abdeckung 26 fortgelassen sind.

Die zweite Ausgestaltung umfasst ein Umsetzungs-Bauteil 41.2, welches an die Stelle des Umsetzungs-Bauteils 41.1 der ersten Ausgestaltung tritt. Die zweite Ausgestaltung unterscheidet sich wie folgt von der ersten Ausgestaltung:
- Bei der ersten Ausgestaltung steht die Mittelachse MA, um welche das Umsetzungs-Bauteil 41.1 drehbar ist, senkrecht auf der Längsachse LA der Stange 4. Bei der zweiten Ausgestaltung verläuft die Mittelachse MA, um welche das Umsetzungs-Bauteil 41.2 drehbar ist, parallel zur Längsachse LA der Stange 4 und weist einen Abstand zu dieser Längsachse LA auf.
- Bei der ersten Ausgestaltung bewegt die Umfangskontur der Nockenscheibe 1 den Stößel 17 und damit die Stange 4. Bei der zweiten Ausgestaltung bewegt hingegen eine Kontur der Nockenscheibe 1, die sich in einer Ebene Eb senkrecht auf der Mittelachse MA erstreckt, den Stößel 17 und damit die Stange 4. Diese Kontur der Nockenscheibe 1 wird im Folgenden als Flächenkontur bezeichnet.
- Bei der ersten Ausgestaltung tritt ein Abstand zwischen der Nockenscheibe 1 und der Lochscheibe 11 auf. Bei der zweiten Ausgestaltung ist die Lochscheibe 11 flächig mit der Nockenscheibe 1 verbunden, und zwar in einer Ebene, die senkrecht auf der Mittelachse MA steht.

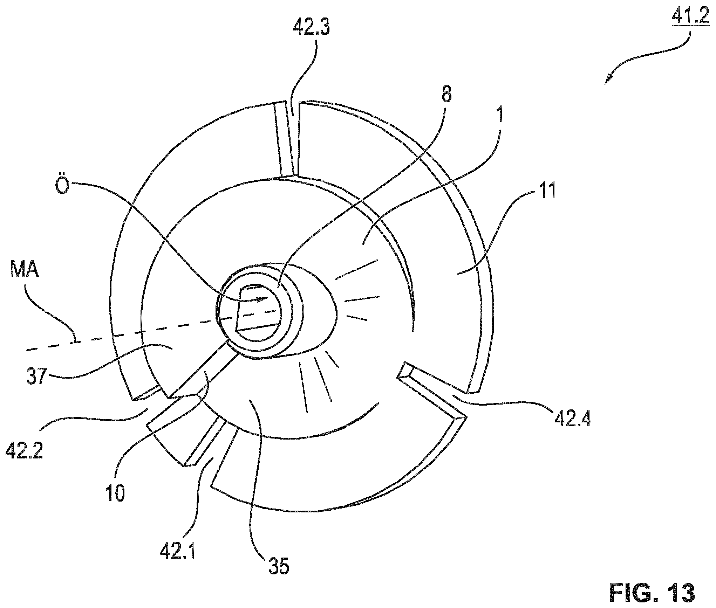

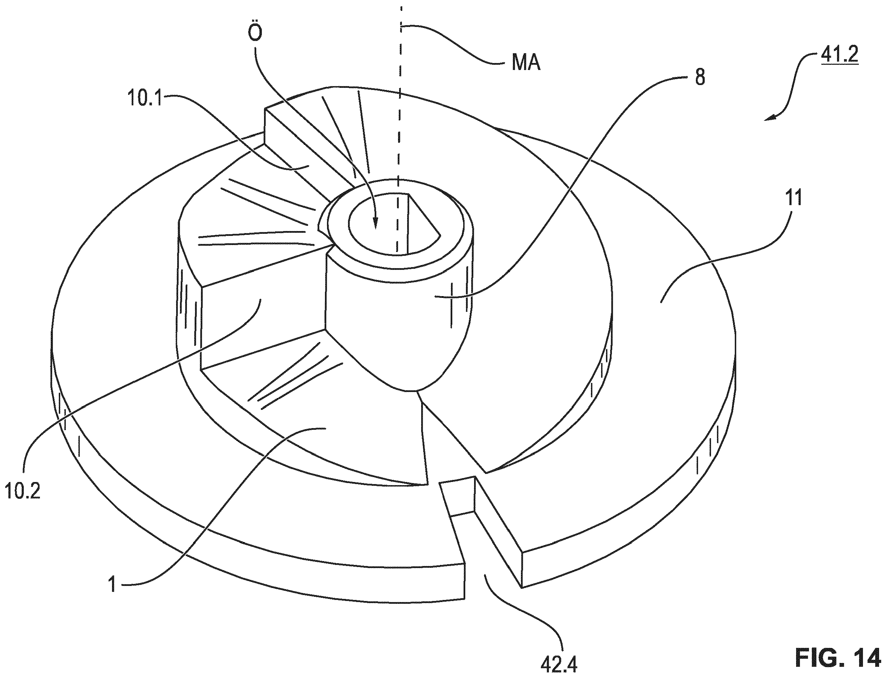

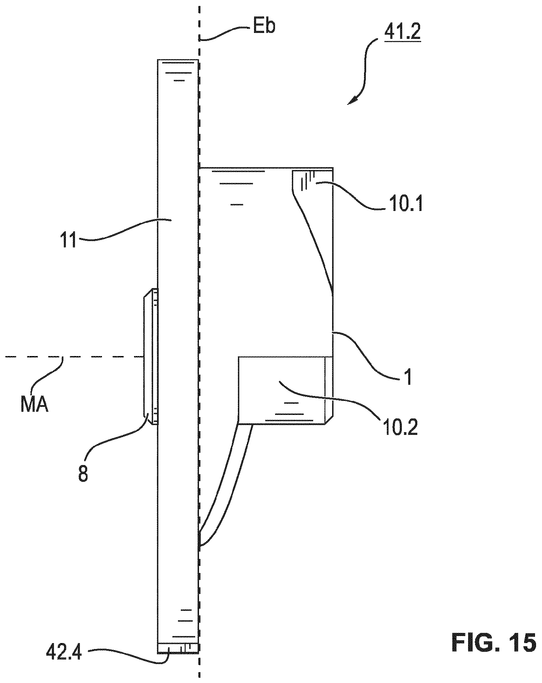

Figur 12 a) und Figur 15 zeigen das Umsetzungs-Bauteil 41.2 aus einer Betrachtungsrichtung senkrecht zur Mittelachse MA. Figur 12 b) zeigt das Umsetzungs-Bauteil 41.2 aus einer Betrachtungsrichtung parallel zur Mittelachse MA, wobei die Nockenscheibe 1 zum Betrachter hin zeigt. Figur 13 und Figur 14 zeigen das Umsetzungs-Bauteil 41.2 in jeweils einer perspektivischen Darstellung. Gleiche Bezugszeichen haben die gleichen Bedeutungen wie bei der ersten Ausgestaltung.

Auch die Flächenkontur umfasst eine Kante 10, 10.1, 10.2 und zwei Segmente 37 und 35. An die Stelle des Radius r gemäß der ersten Ausgestaltung tritt bei der zweiten Ausgestaltung der Abstand der Flächenkontur zu der Referenzebene Eb, die senkrecht auf der Mittelachse MA steht, beispielsweise zu derjenigen Oberfläche der Lochscheibe 11, die zur Nockenscheibe 1 hin zeigt.

Das Umsetzungs-Bauteil 41.2 gemäß der zweiten Ausgestaltung hat eine größere Ausdehnung entlang der Mittelachse MA als das Umsetzungs-Bauteil 41.1 gemäß der ersten Ausgestaltung.

Bei der Realisierungsform gemäß Figur 12 und Figur 13 hat die Nockenscheibe 1 eine Kante 10, bei der Realisierungsform gemäß Figur 14 und Figur 15 zwei Kanten 10.1, 10.2. Diese beiden Realisierungsformen unterscheiden sich voneinander wie folgt:
- Bei der Realisierungsform gemäß Figur 12 und Figur 13 bewirkt eine volle Umdrehung der Nockenscheibe 1 um die Drehachse MA, dass dank der Kante 10 einmal das Volumen der Ansaug-Kammereinheit 5, 6 vergrößert wird und dadurch einmal Gas durch die einströmseitige Fluidverbindung eF hindurch in die Messkammer 3 gesaugt wird.
- Bei der Realisierungsform gemäß Figur 14 und Figur 15 bewirkt eine volle Umdrehung der Nockenscheibe 1 um die Drehachse MA, dass dank der beiden Kanten 10.1 und 10.2 das Volumen der Ansaug-Kammereinheit 5, 6 zweimal vergrößert wird und zwischen den beiden Vergrößerungen wieder verkleinert wird. Dadurch wird zweimal Gas in die Messkammer 3 gesaugt, und zwischendurch wird die Messkammer 3 gespült.

Die Realisierungsform gemäß Figur 14 und Figur 15 ermöglicht, dass von demselben Probanden hintereinander zwei Atemproben eingesaugt und untersucht werden, nämlich zunächst eine Atemprobe, die der Proband zu Beginn des Ausatemvorgangs abgibt, und anschließend eine Atemprobe, dir Proband gegen Ende des Ausatemvorgangs abgibt. Die zuerst abgegebene Atemprobe enthält überwiegend Gas aus dem Mund, die anschließend abgegebene Atemprobe Gas aus der Lunge. Zwischen diesen beiden Ansaug-Vorgängen wird die Messkammer 3 gespült, sodass die beiden Gasproben sich unabhängig voneinander untersuchen lassen.

### Bezugszeichenliste

| | |
|---|---|
| 1 | Nockenscheibe, drehfest mit der Nockenwelle 8 verbunden, hat eine Umfangskontur (erste Ausgestaltung) bzw. eine Flächenkontur (zweite Ausgestaltung), führt den Stößel 17, hat die Kanten 10, 10.1, 10.2, weist den Radius r auf, gehört zum Umsetzungs-Bauteil 41.1, 41.2 |
| 2 | kegelförmiger Ventilkörper, mit einem freien Ende der Stange 4 verbunden, bildet zusammen mit dem Dichtring 13 ein Ventil für die einströmseitige Fluidverbindung eF |
| 3 | Messkammer, nimmt eine zu untersuchende Gasprobe auf, umgibt den Sensor 12, ist von der Wandung 40 umgeben |
| 4 | Stange, erstreckt sich entlang der Längsachse LA, ist an dem einen freien Ende mit dem Ventilkörper 2 und an dem anderen freien Ende mit dem Stößel 17 verbunden |
| 5 | Balg, der einen Unterdruck und einen Überdruck in der Messkammer 3 zu erzeugen vermag, wird von der Stange 4 auseinandergezogen und zusammengedrückt, weist das Volumen Vol auf |
| 6 | Platte, vermag den Balg 5 auseinanderzuziehen und zusammenzudrücken, fest auf der Stange 4 montiert |
| 7 | Elektromotor, dreht über das Untersetzungsgetriebe 25 die Ausgangswelle 36 |
| 8 | Nockenwelle, drehfest mit der Nockenscheibe 1 und der Lochscheibe 11 verbunden, weist die Öffnung Ö auf, durch die hindurch die Ausgangswelle 36 geführt ist, gehört zum Umsetzungs-Bauteil 41.1, 41.2 |
| 9 | Gestell, auf das die übrigen Bestandteile der Gasdetektionsvorrichtung 100 montiert sind |
| 10 | einzige Kante in der Kontur der Nockenscheibe 1 |
| 10.1, 10.2 | Kanten in der Kontur der Nockenscheibe 1 |
| 11 | Lochscheibe (Encoder-Scheibe) , drehfest mit der Nockenwelle 8 verbunden, gehört zum Umsetzungs-Bauteil 41.1, 41.2 und zum Positionssensor |
| 12 | elektrochemischer Sensor in der Messkammer 3, umfasst die Elektroden 20 und 21 sowie die elektrischen Kontaktierungen 33, 34, vermag die Konzentration von Atemalkohol in der Messkammer-Probe Pr zu ermitteln |
| 13 | Dichtring um die Stange 4, fungiert als Ventilkörper-Sitz für den Dichtkegel (Ventilkörper) 2, bildet zusammen mit dem Ventilkörper 2 ein Ventil für die einströmseitige Fluidverbindung eF |
| 14 | weiterer Dichtring, um den Probeneinlass 16 herum angeordnet |
| 15 | signalverarbeitendes Steuergerät (control unit), empfängt ein Signal von der Lichtschranke 23 und steuert den Elektromotor 7 an |
| 16 | Probeneinlass, auf welche sich das Mundstück 30 aufsetzen lässt, stellt einen Teil der einströmseitigen Fluidverbindung eF bereit, wird vom Ventil 2, 13 freigegeben oder verschlossen |
| 17 | Stößel auf einem freien Ende der Stange 4 |
| 18 | Druckfeder, ist bestrebt, die Stange 4 gegen die Umfangskontur (erste Ausgestaltung) bzw. Flächenkontur (zweite Ausgestaltung) der Nockenscheibe 1 zu drücken, stützt sich an der Wandung 40 ab |
| 19 | Verbindungselement zwischen dem Stößel 17 und der Platte 6, umgibt die Stange 4 |
| 20 | Messelektrode des Sensors 12, wird von der elektrischen Kontaktierung 34 kontaktiert |
| 21 | Gegenelektrode des Sensors 12, wird von der elektrischen Kontaktierung 33 kontaktiert |
| 22 | elektrische Verbindung zwischen den Kontaktierungen 33 und 34 |
| 23 | Lichtschranke, erzeugt einen Lichtstrahl, der durch die Lochscheibe 11 unterbrochen und durch die Aussparungen 42.1, 42.2,.com. freigegeben wird, gehört zum Positionssensor |
| 24.1, 24.2 | elektrische Kontaktierungen für die Lichtschranke 23 |
| 25 | Untersetzungsgetriebe zwischen dem Elektromotor 7 und der Ausgangswelle 36 |
| 26 | Abdeckung für das Untersetzungsgetriebe 25 |
| 28 | Elektrolyt zwischen den beiden Elektroden 20 und 21 |
| 29 | elektrischer Messwiderstand zwischen den beiden Elektroden 20, 21 |
| 30 | trichterförmiges Mundstück, leitet eine Gasprobe G in den Probeneinlass 16 |
| 32 | einströmseitiges Anschlussstück, an der Wandung 40 der Messkammer 3 befestigt, stellt einen Teil der einströmseitigen Fluidverbindung eF bereit |
| 33 | elektrische Kontaktierung der Gegenelektrode 21 |
| 34 | elektrische Kontaktierung der Messelektrode 20 |
| 35 | Segment der Umfangskontur der Nockenscheibe 1 mit gleichbleibendem Radius r₁ |
| 36 | Ausgangswelle, wird vom Elektromotor 7 um die Mittelachse MA gedreht, ist durch die Öffnung Ö im Umsetzungs-Bauteil 41.1, 41.2 hindurchgeführt |
| 37 | Segment der Umfangskontur der Nockenscheibe 1 mit gleichbleibendem maximalem Radius rₘₐₓ |
| 38 | Stromstärken-Sensor, misst die Stärke des durch die elektrische Verbindung 22 fließenden Stroms |
| 39 | ausströmseitiges Anschlussstück, an der Wandung 40 der Messkammer 3 befestigt, stellt einen Teil der ausströmseitigen Fluidverbindung aF bereit |
| 40 | Wandung der Messkammer 3, mit dem einströmseitigen Anschlussstück 32 verbunden |
| 41.1 | Umsetzungs-Bauteil gemäß der ersten Ausgestaltung, besteht aus der Nockenwelle 8, der Nockenscheibe 1 und der zur Nockenscheibe 1 beabstandeten Lochscheibe 11, um die Mittelachse MA drehbar, drehfest mit der Ausgangswelle 36 verbunden |
| 41.2 | Umsetzungs-Bauteil gemäß der zweiten Ausgestaltung, besteht aus der Nockenwelle 8, der Nockenscheibe 1 und der mit der Nockenscheibe 1 verbundenen Lochscheibe 11, um die Mittelachse MA drehbar, drehfest mit der Ausgangswelle 36 verbunden |
| 42.1, 42.2, ... | Aussparungen in der Lochscheibe 11, lassen einen Lichtstrahl der Lichtschranke 23 passieren |
| 50 | Sensor-Anordnung, umfasst einen Sensor 12 und eine Messkammer 3 |
| 100 | Analysegerät, umfasst den Sensor 12, die Messkammer 3, den Motor 7, die Ausgangswelle 36, das Umsetzungs-Bauteil 41.1, 41.2, die Stange 4, den Balg 5 und die Platte 6 |
| aF | ausströmseitige Fluidverbindung, verbindet die Messkammer 3 mit dem Balg 5 |
| α | Rotationsposition der Nockenscheibe 1, wird von dem Positionssensor 11, 23 gemessen |
| αₘₐₓ | Rotationsposition, in der die Nockenscheibe 1 den maximalen Radius rₘₐₓ aufweist |
| d | Abstand zwischen dem Ventilkörper 2 und dem Ventilkörper-Sitz 13 |
| dₘₐₓ | maximaler Abstand d, vollständig öffnende Position des Ventils 2, 13 |
| DR | Drehrichtung, in welche der Elektromotor 7 das Umsetzungs-Bauteil 41.1 dreht |
| Eb | Ebene, in welche sich die Flächenkontur der Nockenscheibe 1 erstreckt |
| eF | einströmseitige Fluidverbindung zwischen der Messkammer 3 und dem Mundstück 30 |
| G | Gasprobe, die auf Atemalkohol zu untersuchen ist, enthält die Messkammer-Probe Pr, die in die Messkammer 3 gesaugt wird |
| LA | Längsachse der Stange 4 |
| MA | übereinstimmende Mittelachse der Ausgangswelle 36 und des Umsetzungs-Bauteils 41.1, 41.2 |
| Ö | Öffnung im Umsetzungs-Bauteil 41.1, 41.2, durch welche hindurch die Ausgangswelle 36 geführt ist |
| r | Radius der Nockenscheibe 1, gemessen an der Stelle, an welcher der Stößel 17 die Umfangskontur der Nockenscheibe 1 berührt, variiert entlang der Umfangskontur |
| r₁ | Radius der Nockenscheibe 1, nachdem der Stößel 17 über die Kante 10 geglitten ist |
| rₘᵢₙ | minimaler Radius der Nockenscheibe 1, wird bei der Rotationsposition α=0_{°} erreicht |
| rₘₐₓ | maximaler Radius der Nockenscheibe 1, wird bei der Rotationsposition αₘₐₓ erreicht |
| R.1 | erste Richtung, in welche die Stange 4 beweglich ist, wobei eine Bewegung der Stange in die Richtung R.1 das Volumen Vol des Balgs 5 vergrößert |
| R.2 | zweite Richtung, in welche die Stange 4 beweglich ist, wobei eine Bewegung der Stange in die Richtung R.1 das Volumen Vol des Balgs 5 verkleinert |
| Vol | Volumen des Balgs 5, wird von der Stange 4 verändert |
| Vol₁ | Volumen des Balgs 5, das erzielt wird, nachdem der Stößel 17 über die Kante 10 geglitten ist |
| Volₘₐₓ | maximales Volumen des Balgs 5, wird beim Radius rₘₐₓ erzielt |
| Volₘᵢₙ | minimales Volumen des Balgs 5, wird beim Radius rₘᵢₙ erzielt |

## Patentansprüche

1. Analysegerät (100) zum Analysieren eines Gases (G) auf eine vorgegebene Substanz,
wobei das Analysegerät (100)
- eine Messkammer (3), die wenigstens zeitweise in einer einströmseitigen Fluidverbindung (eF) mit einer Umgebung des Analysegeräts (100) steht,
- einen Sensor (12),
- eine Ansaug-Kammereinheit (5, 6) mit einem veränderbaren Volumen (Vol),
- einen Motor (7),
- eine Nockenscheibe (1), welche um eine Achse (MA) drehbar ist, und
- eine Betätigungseinheit (4, 17, 19), die relativ zur Messkammer (3) in eine erste Richtung (R.1) und eine der ersten Richtung (R.1) entgegengesetzte zweite Richtung (R.2) beweglich ist, bevorzugt linear beweglich ist,
umfasst,
wobei der Sensor (12) dazu ausgestaltet ist, ein Maß für die Menge und / oder die Konzentration der Substanz in einer Gasprobe (Pr), die sich in der Messkammer (3) befindet, zu messen,
wobei die Betätigungseinheit (4, 17, 19) eine Stange (4) und ein Kontaktelement (17) umfasst,
wobei die Betätigungseinheit (4, 17, 19) dergestalt mit der Ansaug-Kammereinheit (5, 6) mechanisch verbunden ist, dass
- eine Bewegung der Betätigungseinheit (4, 17, 19) in die erste Richtung (R.1) das Volumen (Vol) der Ansaug-Kammereinheit (5, 6) vergrößert und
- eine Bewegung der Betätigungseinheit (4, 17, 19) in die zweite Richtung (R.2) das Volumen (Vol) der Ansaug-Kammereinheit (5, 6) verkleinert,
wobei die Ansaug-Kammereinheit (5, 6) dergestalt in einer ausströmseitigen Fluidverbindung (aF) mit der Messkammer (3) steht, dass
- eine Vergrößerung des Volumens (Vol) der Ansaug-Kammereinheit (5, 6) bewirkt, dass Gas (G) aus der Umgebung durch die einströmseitige Fluidverbindung (eF) hindurch in die Messkammer (3) gesaugt wird, und
- eine Verkleinerung des Volumens (Vol) der Ansaug-Kammereinheit (5, 6) bewirkt, dass Gas aus der Messkammer (3) ausgestoßen wird,
wobei der Motor (7) dazu ausgestaltet ist, die Nockenscheibe (1) um die Achse (MA) zu drehen,
wobei die Betätigungseinheit (4, 17, 19) dergestalt mit der Nockenscheibe (1) mechanisch gekoppelt ist,
dass jede Rotationsposition (α) der Nockenscheibe (1) relativ zur Achse (MA) jeweils eine Position der Betätigungseinheit (4, 17, 19) und damit ein Volumen (Vol) der Ansaug-Kammereinheit (5, 6) festlegt, und
wobei die mechanische Kopplung der Betätigungseinheit (4, 17, 19) mit der Nockenscheibe (1) nach Art eines Nockenfolgers ist,
**dadurch gekennzeichnet, dass**
das Analysegerät (100) ein Ventil (2, 13) umfasst, das zwischen einer verschließenden und einer freigebenden Endposition hin und her beweglich ist,
wobei das Ventil (2, 13) in der verschließenden Endposition die einströmseitige Fluidverbindung (eF) verschließt und in der freigebenden Endposition diese freigibt,
wobei das Kontaktelement (17) in Kontakt mit der Nockenscheibe (1) steht und die Stange (4) das Kontaktelement (17) mit dem Ventil (2, 13) verbindet,
wobei entweder
- eine Bewegung der Betätigungseinheit (4, 17, 19) in die erste Richtung (R.1) bewirkt, dass das Ventil (2, 13) in Richtung der verschließenden Endposition bewegt wird, und
- eine Bewegung der Betätigungseinheit (4, 17, 19) in die zweite Richtung (R.2) bewirkt, dass das Ventil (2, 13) in Richtung der freigebenden Endposition bewegt wird,
oder
- eine Bewegung der Betätigungseinheit (4, 17, 19) in die erste Richtung (R.1) bewirkt, dass das Ventil (2, 13) in Richtung der freigebenden Endposition bewegt wird, und
- eine Bewegung der Betätigungseinheit (4, 17, 19) in die zweite Richtung (R.2) bewirkt, dass das Ventil (2, 13) in Richtung der verschließenden Endposition bewegt wird.

2. Analysegerät (100) nach Anspruch 1,
**dadurch gekennzeichnet, dass**
eine erste Rotationsposition (rₘᵢₙ) der Nockenscheibe (1) ein maximales Volumen (Volₘₐₓ) und eine zweite Rotationsposition (rₘₐₓ) der Nockenscheibe (1) ein minimales Volumen (Volₘᵢₙ) der Ansaug-Kammereinheit (5, 6) festlegen.

3. Analysegerät (100) nach einem der vorhergehenden Ansprüche,
**dadurch gekennzeichnet, dass**
das Analysegerät (100) eine Feder (18) umfasst,
wobei die Feder (18) bestrebt ist, die Betätigungseinheit (4, 17, 19) gegen die Nockenscheibe (1) zu drücken und dadurch die mechanische Kopplung herzustellen oder wenigstens zu dieser beizutragen.

4. Analysegerät (100) nach einem der vorhergehenden Ansprüche,
**dadurch gekennzeichnet, dass**
die Nockenscheibe (1) eine Umfangskontur aufweist, die sich um die Achse (MA) herum erstreckt,
wobei der Abstand (r) zwischen der Umfangskontur und der Achse (MA) entlang der Umfangskontur variiert, und
die beiden Richtungen (R.1, R.2), in welche die Betätigungseinheit (4, 17, 19) beweglich ist, senkrecht auf der Achse (MA) stehen,
wobei die Betätigungseinheit (4, 17, 19) in Kontakt mit der Umfangskontur steht und
wobei bevorzugt eine Feder (18) bestrebt ist, die Betätigungseinheit (4, 17, 19) gegen die Umfangskontur zu drücken.

5. Analysegerät (100) nach einem der Ansprüche 1 bis 3,
**dadurch gekennzeichnet, dass**
die Nockenscheibe (1) eine Flächenkontur aufweist, die sich in einer Ebene (Eb) erstreckt, die senkrecht auf der Achse (MA) steht,
wobei der Abstand zwischen der Flächenkontur und dieser Ebene (Eb) über die Flächenkontur variiert, und
die beiden Richtungen (R.1, R.2), in welche die Betätigungseinheit (4, 17, 19) beweglich ist, parallel zur Achse (MA) sind,
wobei die Betätigungseinheit (4, 17, 19) in Kontakt mit der Flächenkontur steht und
wobei bevorzugt eine Feder (18) bestrebt ist, die Betätigungseinheit (4, 17, 19) gegen die Flächenkontur zu drücken.

6. Analysegerät (100) nach einem der vorhergehenden Ansprüche,
**dadurch gekennzeichnet, dass**
die Nockenscheibe (1), die Betätigungseinheit (4, 17, 19) und die Messkammer (3) in einer Linie liegen.

7. Analysegerät (100) nach einem der vorhergehenden Ansprüche,
**dadurch gekennzeichnet, dass**
die Nockenscheibe (1) so ausgestaltet ist,
dass eine volle Umdrehung der Nockenscheibe (1) um die Achse (MA) bewirkt, dass die Betätigungseinheit (4, 17, 19)
- mindestens zweimal hintereinander in die erste Richtung (R.1) und
- mindestens einmal in die zweite Richtung (R.2)
bewegt wird,
wobei die Bewegung in die zweite Richtung (R.2) zwischen den beiden Bewegungen in die erste Richtung (R.1) stattfindet.

8. Analysegerät (100) nach einem der vorhergehenden Ansprüche,
**dadurch gekennzeichnet, dass**
das Analysegerät (100) einen Positionssensor (11, 23) umfasst,
wobei der Positionssensor (11, 23) dazu ausgestaltet ist, ein Maß für die Rotationsposition (α) der Nockenscheibe (1) relativ zur Achse (MA) zu messen.

9. Analysegerät (100) nach Anspruch 8,
**dadurch gekennzeichnet, dass**
das Analysegerät (100) ein signalverarbeitendes Steuergerät (15) umfasst und
der Motor (7) aktivierbar und deaktivierbar ist,
wobei das Steuergerät (15) dazu ausgestaltet ist,
- ein Signal des Positionssensors (11, 23) zu empfangen und
- den Motor (7) abhängig von dem Signal des Positionssensors (11, 23) zu aktivieren und wieder zu deaktivieren.

10. Analysegerät (100) nach Anspruch 8 oder Anspruch 9,
**dadurch gekennzeichnet, dass**
der Positionssensor (11, 23) eine Lochscheibe (11) und eine Lichtschranke (23) umfasst,
wobei die Lochscheibe (11) drehfest mit der Nockenscheibe (1) verbunden ist und die Lochscheibe (11) einen Lichtstrahl der Lichtschranke (23) freizugeben oder zu unterbrechen vermag.

11. Analyseverfahren zum Analysieren eines Gases (G) auf eine vorgegebene Substanz
unter Verwendung eines Analysegeräts (100), das
- eine Messkammer (3), die wenigstens zeitweise in einer einströmseitigen Fluidverbindung (eF) mit einer Umgebung des Analysegeräts (100) steht,
- einen Sensor (12),
- eine Ansaug-Kammereinheit (5, 6) mit einem veränderbaren Volumen (Vol),
- einen Motor (7),
- eine Nockenscheibe (1), welche um eine Achse (MA) drehbar ist, und
- eine Betätigungseinheit (4, 17, 19), die relativ zur Messkammer (3) in eine erste Richtung (R.1) und eine der ersten Richtung (R.1) entgegengesetzte zweite Richtung (R.2) beweglich ist, bevorzugt linear beweglich ist,
umfasst,
wobei die Betätigungseinheit (4, 17, 19) eine Stange (4) und ein Kontaktelement (17) umfasst,
wobei die mechanische Kopplung der Betätigungseinheit (4, 17, 19) mit der Nockenscheibe (1) nach Art eines Nockenfolgers ist und
wobei das Verfahren die Schritte umfasst, dass
- der Motor (7) wenigstens zeitweise die Nockenscheibe (1) um die Achse (MA) dreht,
- eine mechanische Kopplung zwischen der Nockenscheibe (1) und der Betätigungseinheit (4, 17, 19) bewirkt, dass jede Rotationsposition (α) der Nockenscheibe (1) relativ zur Achse (MA) jeweils eine Position der Betätigungseinheit (4, 17, 19) und damit jeweils ein Volumen (Vol) der Ansaug-Kammereinheit (5, 6) festlegt,
- eine Bewegung der Betätigungseinheit (4, 17, 19) in die erste Richtung (R.1) das Volumen (Vol) der Ansaug-Kammereinheit (5, 6) vergrößert,
- die Vergrößerung des Volumens (Vol) der Ansaug-Kammereinheit (5, 6) bewirkt, dass Gas (G) aus der Umgebung durch die einströmseitige Fluidverbindung (eF) hindurch in die Messkammer (3) gesaugt wird,
- der Sensor (12) ein Maß für die Menge und / oder die Konzentration der Substanz in einer Gasprobe (Pr), die sich in der Messkammer (3) befindet, misst,
- eine Bewegung der Betätigungseinheit (4, 17, 19) in die zweite Richtung (R.2) das Volumen (Vol) der Ansaug-Kammereinheit (5, 6) verkleinert und
- die Verkleinerung des Volumens (Vol) der Ansaug-Kammereinheit (5, 6) bewirkt, dass Gas aus der Messkammer (3) ausgestoßen wird,
**dadurch gekennzeichnet, dass**
das Analysegerät (100) ein Ventil (2, 13) umfasst, das zwischen einer verschließenden und einer freigebenden Endposition hin und her beweglich ist,
wobei das Ventil (2, 13) in der verschließenden Endposition die einströmseitige Fluidverbindung (eF) verschließt und in der freigebenden Endposition diese freigibt,
wobei das Kontaktelement (17) in Kontakt mit der Nockenscheibe (1) steht und die Stange (4) das Kontaktelement (17) mit dem Ventil (2, 13) verbindet,
wobei entweder
- eine Bewegung der Betätigungseinheit (4, 17, 19) in die erste Richtung (R.1) bewirkt, dass das Ventil (2, 13) in Richtung der verschließenden Endposition bewegt wird, und
- eine Bewegung der Betätigungseinheit (4, 17, 19) in die zweite Richtung (R.2) bewirkt, dass das Ventil (2, 13) in Richtung der freigebenden Endposition bewegt wird,
oder
- eine Bewegung der Betätigungseinheit (4, 17, 19) in die erste Richtung (R.1) bewirkt, dass das Ventil (2, 13) in Richtung der freigebenden Endposition bewegt wird, und
- eine Bewegung der Betätigungseinheit (4, 17, 19) in die zweite Richtung (R.2) bewirkt, dass das Ventil (2, 13) in Richtung der verschließenden Endposition bewegt wird.

## Claims

1. Analyzer (100) for analyzing a gas (G) for a specified substance,
wherein the analyzer (100) comprises
- a measuring chamber (3) which is at least temporarily in an inflow-side fluid connection (eF) to surroundings of the analyzer (100),
- a sensor (12),
- a suction chamber unit (5, 6) having a variable volume (Vol),
- a motor (7),
- a cam disk (1) which is rotatable about an axis (MA), and
- an actuation unit (4, 17, 19) which is movable, preferably linearly movable, relative to the measuring chamber (3) in a first direction (R.1) and a second direction (R.2) counter to the first direction (R.1),
wherein the sensor (12) is configured to measure an indicator for the amount and/or the concentration of the substance in a gas sample (Pr) located in the measuring chamber (3),
wherein the actuation unit (4, 17, 19) comprises a rod (4) and a contact element (17),
wherein the actuation unit (4, 17, 19) is mechanically connected to the suction chamber unit (5, 6) such that
- a movement of the actuation unit (4, 17, 19) in the first direction (R.1) increases the volume (Vol) of the suction chamber unit (5, 6) and
- a movement of the actuation unit (4, 17, 19) in the second direction (R.2) reduces the volume (Vol) of the suction chamber unit (5, 6),
wherein the suction chamber unit (5, 6) is in an outflow-side fluid connection (aF) to the measuring chamber (3) such that
- an increase in the volume (Vol) of the suction chamber unit (5, 6) causes gas (G) from the surroundings to be sucked through the inflow-side fluid connection (eF) into the measuring chamber (3), and
- a reduction in the volume (Vol) of the suction chamber unit (5, 6) causes gas to be expelled from the measuring chamber (3),
wherein the motor (7) is configured to rotate the cam disk (1) about the axis (MA),
wherein the actuation unit (4, 17, 19) is mechanically coupled to the cam disk (1) such that
each rotational position (α) of the cam disk (1) relative to the axis (MA) defines in each case a position of the actuation unit (4, 17, 19) and therefore a volume (Vol) of the suction chamber unit (5, 6), and
wherein the mechanical coupling of the actuation unit (4, 17, 19) to the cam disk (1) is in the manner of a cam follower,
**characterized in that**
the analyzer (100) comprises a valve (2, 13) which is movable back and forth between a closing and an opening end position,
wherein the valve (2, 13) closes the inflow-side fluid connection (eF) in the closing end position and opens it in the opening end position,
wherein the contact element (17) is in contact with the cam disk (1) and the rod (4) connects the contact element (17) to the valve (2, 13),
wherein either
- a movement of the actuation unit (4, 17, 19) in the first direction (R.1) causes the valve (2, 13) to be moved towards the closing end position, and
- a movement of the actuation unit (4, 17, 19) in the second direction (R.2) causes the valve (2, 13) to be moved towards the opening end position,
or
- a movement of the actuation unit (4, 17, 19) in the first direction (R.1) causes the valve (2, 13) to be moved towards the opening end position, and
- a movement of the actuation unit (4, 17, 19) in the second direction (R.2) causes the valve (2, 13) to be moved towards the closing end position.

2. Analyzer (100) according to claim 1,
**characterized in that**
a first rotational position (rₘᵢₙ) of the cam disk (1) defines a maximum volume (Volₘₐₓ) and a second rotational position (rₘₐₓ) of the cam disk (1) defines a minimum volume (Volₘᵢₙ) of the suction chamber unit (5, 6).

3. Analyzer (100) according to either of the preceding claims,
**characterized in that**
the analyzer (100) comprises a spring (18),
wherein the spring (18) is intended to press the actuation unit (4, 17, 19) against the cam disk (1) and thereby to establish or at least contribute to the mechanical coupling.

4. Analyzer (100) according to any of the preceding claims,
**characterized in that**
the cam disk (1) has a circumferential contour which extends around the axis (MA),
wherein the distance (r) between the circumferential contour and the axis (MA) varies around the circumferential contour, and
the two directions (R.1, R.2) in which the actuation unit (4, 17, 19) is movable are perpendicular to the axis (MA),
wherein the actuation unit (4, 17, 19) is in contact with the circumferential contour and
wherein preferably a spring (18) is intended to press the actuation unit (4, 17, 19) against the circumferential contour.

5. Analyzer (100) according to any of claims 1 to 3,
**characterized in that**
the cam disk (1) has a surface contour which extends in a plane (Eb) which is perpendicular to the axis (MA),
wherein the distance between the surface contour and this plane (Eb) varies across the surface contour, and
the two directions (R.1, R.2) in which the actuation unit (4, 17, 19) is movable are parallel to the axis (MA),
wherein the actuation unit (4, 17, 19) is in contact with the surface contour and
wherein preferably a spring (18) is intended to press the actuation unit (4, 17, 19) against the surface contour.

6. Analyzer (100) according to any of the preceding claims,
**characterized in that**
the cam disc (1), the actuation unit (4, 17, 19) and the measuring chamber (3) are in a line.

7. Analyzer (100) according to any of the preceding claims,
**characterized in that**
the cam disc (1) is configured such that
one full revolution of the cam disc (1) about the axis (MA) causes the actuation unit (4, 17, 19) to be moved
- at least twice in succession in the first direction (R.1) and
- at least once in the second direction (R.2),
wherein the movement in the second direction (R.2) takes place between the two movements in the first direction (R.1).

8. Analyzer (100) according to any of the preceding claims,
**characterized in that**
the analyzer (100) comprises a position sensor (11, 23),
wherein the position sensor (11, 23) is configured to measure an indicator for the rotational position (α) of the cam disk (1) relative to the axis (MA).

9. Analyzer (100) according to claim 8,
**characterized in that**
the analyzer (100) comprises a signal processing controller (15) and
the motor (7) can be activated and deactivated,
wherein the controller (15) is configured
- to receive a signal from the position sensor (11, 23) and
- to activate and deactivate the motor (7) again depending on the signal from the position sensor (11, 23).

10. Analyzer (100) according to claim 8 or claim 9,
**characterized in that**
the position sensor (11, 23) comprises a perforated disk (11) and a photoelectric sensor (23),
wherein the perforated disk (11) is connected for conjoint rotation to the cam disk (1) and the perforated disk (11) is able to unblock or to interrupt a light beam of the photoelectric sensor (23).

11. Analysis method for analyzing a gas (G) for a specific substance
using an analyzer (100) which comprises
- a measuring chamber (3) which is at least temporarily in an inflow-side fluid connection (eF) to surroundings of the analyzer (100),
- a sensor (12),
- a suction chamber unit (5, 6) having a variable volume (Vol),
- a motor (7),
- a cam disk (1) which is rotatable about an axis (MA), and
- an actuation unit (4, 17, 19) which is movable, preferably linearly movable, relative to the measuring chamber (3) in a first direction (R.1) and a second direction (R.2) counter to the first direction (R.1),
wherein the actuation unit (4, 17, 19) comprises a rod (4) and a contact element (17),
wherein the mechanical coupling of the actuation unit (4, 17, 19) to the cam disk (1) is in the manner of a cam follower and
wherein the method comprises the steps of
- the motor (7) at least temporarily rotating the cam disk (1) about the axis (MA),
- a mechanical coupling between the cam disk (1) and the actuation unit (4, 17, 19) causing each rotational position (α) of the cam disk (1) relative to the axis (MA) to define in each case a position of the actuation unit (4, 17, 19) and therefore in each case a volume (Vol) of the suction chamber unit (5, 6),
- a movement of the actuation unit (4, 17, 19) in the first direction (R.1) increasing the volume (Vol) of the suction chamber unit (5, 6),
- the increase in the volume (Vol) of the suction chamber unit (5, 6) causing gas (G) from the surroundings to be sucked through the inflow-side fluid connection (eF) into the measuring chamber (3),
- the sensor (12) measuring an indicator for the amount and/or the concentration of the substance in a gas sample (Pr) located in the measuring chamber (3),
- a movement of the actuation unit (4, 17, 19) in the second direction (R.2) reducing the volume (Vol) of the suction chamber unit (5, 6) and
- the reduction of the volume (Vol) of the suction chamber unit (5, 6) causing gas to be expelled from the measuring chamber (3),
**characterized in that**
the analyzer (100) comprises a valve (2, 13) which is movable back and forth between a closing and an opening end position,
wherein the valve (2, 13) closes the inflow-side fluid connection (eF) in the closing end position and opens it in the opening end position,
wherein the contact element (17) is in contact with the cam disk (1) and the rod (4) connects the contact element (17) to the valve (2, 13), wherein either
- a movement of the actuation unit (4, 17, 19) in the first direction (R.1) causes the valve (2, 13) to be moved towards the closing end position, and
- a movement of the actuation unit (4, 17, 19) in the second direction (R.2) causes the valve (2, 13) to be moved towards the opening end position,
or
- a movement of the actuation unit (4, 17, 19) in the first direction (R.1) causes the valve (2, 13) to be moved towards the opening end position, and
- a movement of the actuation unit (4, 17, 19) in the second direction (R.2) causes the valve (2, 13) to be moved towards the closing end position.

## Revendications

1. Appareil d'analyse (100) permettant d'analyser un gaz (G) à la recherche d'une substance prédéfinie,
dans lequel l'appareil d'analyse (100) comprend
- une chambre de mesure (3) qui est, au moins temporairement, en liaison fluidique côté admission (eF) avec un environnement de l'appareil d'analyse (100),
- un capteur (12),
- une unité de chambre d'aspiration (5, 6) à volume (Vol) variable,
- un moteur (7),
- un disque à cames (1) qui peut tourner autour d'un axe (MA), et
- une unité d'actionnement (4, 17, 19) qui peut se déplacer par rapport à la chambre de mesure (3) dans une première direction (R.1) et dans une seconde direction (R.2) opposée à la première direction (R.1), de préférence de manière linéaire,
dans lequel le capteur (12) est configuré pour mesurer une mesure de la quantité et/ou de la concentration de la substance dans un échantillon de gaz (Pr) qui se trouve dans la chambre de mesure (3),
dans lequel l'unité d'actionnement (4, 17, 19) comprend une tige (4) et un élément de contact (17),
dans lequel l'unité d'actionnement (4, 17, 19) est reliée mécaniquement à l'unité de chambre d'aspiration (5, 6) de telle sorte
- qu'un déplacement de l'unité d'actionnement (4, 17, 19) dans la première direction (R. 1) augmente le volume (Vol) de l'unité de chambre d'aspiration (5, 6) et
- qu'un déplacement de l'unité d'actionnement (4, 17, 19) dans la seconde direction (R.2) réduit le volume (Vol) de l'unité de chambre d'aspiration (5, 6), dans lequel l'unité de chambre d'aspiration (5, 6) est en liaison fluidique côté éjection (aF) avec la chambre de mesure (3) de telle sorte
- qu'une augmentation du volume (Vol) de l'unité de chambre d'aspiration (5, 6) provoque l'aspiration de gaz (G) provenant de l'environnement à travers la liaison fluidique côté admission (eF) vers la chambre de mesure (3), et
- qu'une réduction du volume (Vol) de l'unité de chambre d'aspiration (5, 6) provoque l'expulsion du gaz hors de la chambre de mesure (3),
dans lequel le moteur (7) est configuré pour faire tourner le disque à cames (1) autour de l'axe (MA),
dans lequel l'unité d'actionnement (4, 17, 19) est couplée mécaniquement au disque à cames (1) de telle sorte
que chaque position de rotation (α) du disque à cames (1) par rapport à l'axe (MA) établit respectivement une position de l'unité d'actionnement (4, 17, 19) et donc un volume (Vol) de l'unité de chambre d'aspiration (5, 6), et
dans lequel le couplage mécanique entre l'unité d'actionnement (4, 17, 19) et le disque à cames (1) s'effectue à la manière d'un suiveur de came,
**caractérisé en ce que**
l'appareil d'analyse (100) comprend une vanne (2, 13) qui peut se déplacer en va-et-vient entre une position finale de fermeture et une position finale de libération,
dans lequel la vanne (2, 13) ferme, dans sa position finale de fermeture, la liaison fluidique côté admission (eF) et la libère dans sa position finale de libération,
dans lequel l'élément de contact (17) est en contact avec le disque à cames (1) et la tige (4) relie l'élément de contact (17) à la vanne (2, 13),
dans lequel soit
- un déplacement de l'unité d'actionnement (4, 17, 19) dans la première direction (R.1) provoque le déplacement de la vanne (2, 13) vers la position finale de fermeture, et
- un déplacement de l'unité d'actionnement (4, 17, 19) dans la seconde direction (R.2) provoque le déplacement de la vanne (2, 13) vers la position finale de libération,
soit
- un déplacement de l'unité d'actionnement (4, 17, 19) dans la première direction (R.1) provoque le déplacement de la vanne (2, 13) vers la position finale de libération, et
- un déplacement de l'unité d'actionnement (4, 17, 19) dans la seconde direction (R.2) provoque le déplacement de la vanne (2, 13) vers la position finale de fermeture.

2. Appareil d'analyse (100) selon la revendication 1,
**caractérisé en ce**
**qu'**une première position de rotation (rₘᵢₙ) du disque à cames (1) établit un volume maximal (Volₘₐₓ) et une seconde position de rotation (rₘₐₓ) du disque à cames (1) établit un volume minimal (Volₘᵢₙ) de l'unité de chambre d'aspiration (5, 6).

3. Appareil d'analyse (100) selon l'une des revendications précédentes,
**caractérisé en ce que**
l'appareil d'analyse (100) comprend un ressort (18),
dans lequel le ressort (18) tend à pousser l'unité d'actionnement (4, 17, 19) contre le disque à cames (1) et à créer ainsi le couplage mécanique, ou du moins à y contribuer.

4. Appareil d'analyse (100) selon l'une des revendications précédentes,
**caractérisé en ce que**
le disque à cames (1) présente un contour périphérique qui s'étend autour de l'axe (MA),
dans lequel la distance (r) entre le contour périphérique et l'axe (MA) varie le long du contour périphérique, et
les deux directions (R.1, R.2) dans lesquelles l'unité d'actionnement (4, 17, 19) peut se déplacer sont perpendiculaires à l'axe (MA),
dans lequel l'unité d'actionnement (4, 17, 19) est en contact avec le contour périphérique et
dans lequel un ressort (18) tend de préférence à pousser l'unité d'actionnement (4, 17, 19) contre le contour périphérique.

5. Appareil d'analyse (100) selon l'une des revendications 1 à 3,
**caractérisé en ce que**
le disque à cames (1) présente un profil de surface qui s'étend dans un plan (Eb) qui est perpendiculaire à l'axe (MA),
dans lequel la distance entre le contour de surface et ce plan (Eb) varie le long du contour de surface, et
les deux directions (R.1, R.2) dans lesquelles l'unité d'actionnement (4, 17, 19) peut se déplacer sont parallèles à l'axe (MA),
dans lequel l'unité d'actionnement (4, 17, 19) est en contact avec le contour de surface et
dans lequel un ressort (18) tend de préférence à pousser l'unité d'actionnement (4, 17, 19) contre le contour de surface.

6. Appareil d'analyse (100) selon l'une des revendications précédentes,
**caractérisé en ce que**
le disque à cames (1), l'unité d'actionnement (4, 17, 19) et la chambre de mesure (3) se trouvent sur une ligne.

7. Appareil d'analyse (100) selon l'une des revendications précédentes,
**caractérisé en ce que**
le disque à cames (1) est configuré de telle sorte
qu'un tour complet du disque à cames (1) autour de l'axe (MA) provoque le déplacement de l'unité d'actionnement (4, 17, 19)
- au moins deux fois de suite dans la première direction (R.1) et
- au moins une fois dans la seconde direction (R.2),
dans lequel le déplacement dans la seconde direction (R.2) a lieu entre les deux déplacements dans la première direction (R.1).

8. Appareil d'analyse (100) selon l'une des revendications précédentes,
**caractérisé en ce que**
l'appareil d'analyse (100) comprend un capteur de position (11, 23),
dans lequel le capteur de position (11, 23) est configuré pour mesurer une mesure pour la position de rotation (α) du disque à cames (1) par rapport à l'axe (MA).

9. Appareil d'analyse (100) selon la revendication 8,
**caractérisé en ce que**
l'appareil d'analyse (100) comprend un appareil de commande (15) de traitement de signaux et
le moteur (7) peut être activé et désactivé,
dans lequel l'appareil de commande (15) est configuré pour
- recevoir un signal provenant du capteur de position (11, 23) et
- activer puis désactiver de nouveau le moteur (7) en fonction du signal émis par le capteur de position (11, 23).

10. Appareil d'analyse (100) selon la revendication 8 ou la revendication 9,
**caractérisé en ce que**
le capteur de position (11, 23) comprend un disque perforé (11) et une barrière photoélectrique (23),
dans lequel le disque perforé (11) est relié solidaire en rotation avec le disque à cames (1), et le disque perforé (11) peut laisser passer ou interrompre un faisceau lumineux provenant de la barrière photoélectrique (23).

11. Procédé d'analyse permettant d'analyser un gaz (G) à la recherche d'une substance prédéfinie, à l'aide d'un appareil d'analyse (100) qui comprend
- une chambre de mesure (3) qui est, au moins temporairement, en liaison fluidique côté admission (eF) avec un environnement de l'appareil d'analyse (100),
- un capteur (12),
- une unité de chambre d'aspiration (5, 6) à volume (Vol) variable,
- un moteur (7),
- un disque à cames (1) qui peut tourner autour d'un axe (MA), et
- une unité d'actionnement (4, 17, 19) qui peut se déplacer par rapport à la chambre de mesure (3) dans une première direction (R.1) et dans une seconde direction (R.2) opposée à la première direction (R.1), de préférence de manière linéaire,
dans lequel l'unité d'actionnement (4, 17, 19) comprend une tige (4) et un élément de contact (17),
dans lequel le couplage mécanique entre l'unité d'actionnement (4, 17, 19) et le disque à cames (1) s'effectue à la manière d'un suiveur de came et
dans lequel le procédé comprend les étapes selon lesquelles
- le moteur (7) fait tourner, au moins temporairement, le disque à cames (1) autour de l'axe (MA),
- un couplage mécanique entre le disque à cames (1) et l'unité d'actionnement (4, 17, 19) provoque que chaque position de rotation (α) du disque à cames (1) par rapport à l'axe (MA) établit respectivement une position de l'unité d'actionnement (4, 17, 19) et, donc respectivement un volume (Vol) de l'unité de chambre d'aspiration (5, 6),
- un déplacement de l'unité d'actionnement (4, 17, 19) dans la première direction (R.1) augmente le volume (Vol) de l'unité de chambre d'aspiration (5, 6),
- l'augmentation du volume (Vol) de l'unité de chambre d'aspiration (5, 6) provoque l'aspiration du gaz (G) provenant de l'environnement à travers la liaison fluidique côté admission (eF) vers la chambre de mesure (3),
- le capteur (12) mesure une mesure de la quantité et/ou de la concentration de la substance dans un échantillon de gaz (Pr) qui se trouve dans la chambre de mesure (3),
- un déplacement de l'unité d'actionnement (4, 17, 19) dans la seconde direction (R.2) réduit le volume (Vol) de l'unité de chambre d'aspiration (5, 6) et
- la réduction du volume (Vol) de l'unité de chambre d'aspiration (5, 6) provoque l'expulsion du gaz hors de la chambre de mesure (3),
**caractérisé en ce que**
l'appareil d'analyse (100) comprend une vanne (2, 13) qui peut se déplacer en va-et-vient entre une position finale de fermeture et une position finale de libération,
dans lequel la vanne (2, 13) ferme, dans sa position finale de fermeture, la liaison fluidique côté admission (eF) et la libère dans sa position finale de libération,
dans lequel l'élément de contact (17) est en contact avec le disque à cames (1) et la tige (4) relie l'élément de contact (17) à la vanne (2, 13), dans lequel soit
- un déplacement de l'unité d'actionnement (4, 17, 19) dans la première direction (R.1) provoque le déplacement de la vanne (2, 13) vers la position finale de fermeture, et
- un déplacement de l'unité d'actionnement (4, 17, 19) dans la seconde direction (R.2) provoque le déplacement de la vanne (2, 13) vers la position finale de libération,
soit
- un déplacement de l'unité d'actionnement (4, 17, 19) dans la première direction (R.1) provoque le déplacement de la vanne (2, 13) vers la position finale de libération, et
- un déplacement de l'unité d'actionnement (4, 17, 19) dans la seconde direction (R.2) provoque le déplacement de la vanne (2, 13) vers la position finale de fermeture.
